# EUROPEAN PATENT APPLICATION

(11) **EP 4 332 096 A1**
(43) Date of publication of application: **06.03.2024**
(21) Application number: 23193103.1
(22) Date of filing: 24.08.2023
(51) Int. Cl.: C07D 333/50, C07D 409/10, H10K 50/11, H10K 85/60

(54) **ORGANIC COMPOUND AND ORGANIC LIGHT-EMITTING ELEMENT**

(30) Priority: 29.08.2022 JP 2022136001
(71) Applicant: CANON KABUSHIKI KAISHA, Tokyo 146-8501 (JP)
(72) Inventor: IWAWAKI, Hironobu, Tokyo 146-8501 (JP); OHRUI, Hiroki, Tokyo 146-8501 (JP); NISHIDE, Yosuke, Tokyo 146-8501 (JP); MIYASHITA, Hirokazu, Tokyo 146-8501 (JP); HORIUCHI, Takayuki, Tokyo 146-8501 (JP); YAMADA, Naoki, Tokyo 146-8501 (JP)
(74) Representative: TBK

(57) **Abstract**

The present disclosure discloses an organic compound represented by the following general formula [1]:

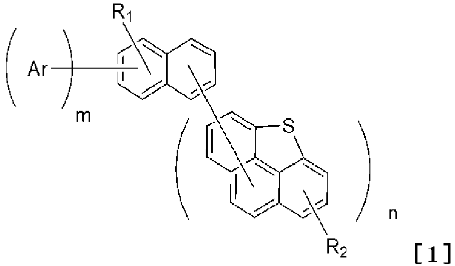

Ar is selected from the group consisting of a substituted or unsubstituted aryl group and a substituted or unsubstituted heteroaryl group. R₁ and R₂ are independently selected from the group consisting of a hydrogen atom and a substituent. m and n are selected from integers of 1 or more and 3 or less.

## Description

### BACKGROUND OF THE INVENTION

### Field of the Invention

The present disclosure relates to an organic compound and an organic light-emitting element including the organic compound.

### Description of the Related Art

An organic light-emitting element (hereinafter sometimes referred to as an "organic electroluminescent element" or an "organic EL element") is an electronic element that includes a pair of electrodes and an organic compound layer between the electrodes. Electrons and holes are injected from the pair of electrodes to generate an exciton of a light-emitting organic compound in the organic compound layer. When the exciton returns to its ground state, the organic light-emitting element emits light.

With recent significant advances in organic light-emitting elements, it is characteristically possible to realize low drive voltage, various emission wavelengths, high-speed responsivity, and thin and light light-emitting devices.

With respect to an increase in efficiency, an element containing an efficiency-improving material, such as a phosphorescent material or a delayed fluorescent material, can be mentioned. In an organic light-emitting element containing such an efficiency-improving material, improvement of the durability performance of the element is a development subject. To date, various improvement methods have been proposed as methods for improving element durability performance, and one of them is stabilization of a material structure constituting a light-emitting layer. Korean Patent Laid-Open No. 2014-091487 describes the following compound 1-A.

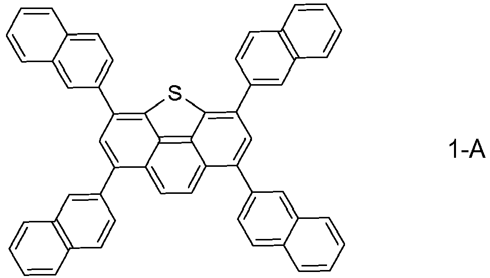

When a compound described in Patent Literature 1 is used for an organic light-emitting element, the organic light-emitting element cannot have high durability.

### SUMMARY OF THE INVENTION

The present disclosure provides an organic compound that can be used for an organic light-emitting element with good element life characteristics.

The present invention in its first aspect provides an organic compound as specified in claims 1 to 5.

The present invention in its second aspect provides an organic light-emitting element as specified in claims 6 to 15.

The present invention in its third aspect provides a display apparatus as specified in claim 16.

The present invention in its fourth aspect provides a photoelectric conversion apparatus as specified in claim 17.

The present invention in its fifth aspect provides electronic equipment as specified in claim 18.

The present invention in its sixth aspect provides a lighting apparatus as specified in claim 19.

The present invention in its seventh aspect provides a moving body as specified in claim 20.

The present invention in its seventh aspect provides an exposure light source as specified in claim 21.

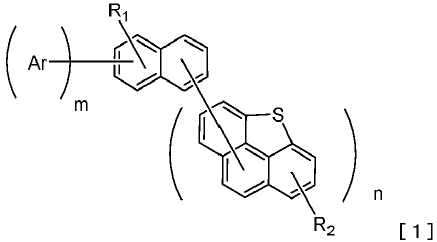

Further features of the present invention will become apparent from the following description of exemplary embodiments with reference to the attached drawings.

### BRIEF DESCRIPTION OF THE DRAWINGS

Fig. 1A is a schematic cross-sectional view of an example of a pixel of a display apparatus according to an embodiment of the present disclosure.
Fig. 1B is a schematic cross-sectional view of an example of a display apparatus including an organic light-emitting element according to an embodiment of the present disclosure.
Fig. 2 is a schematic view of an example of a display apparatus according to an embodiment of the present disclosure.
Fig. 3A is a schematic view of an example of an imaging apparatus according to an embodiment of the present disclosure.
Fig. 3B is a schematic view of an example of electronic equipment according to an embodiment of the present disclosure.
Fig. 4Ais a schematic view of an example of a display apparatus according to an embodiment of the present disclosure.
Fig. 4B is a schematic view of an example of a foldable display apparatus.
Fig. 5A is a schematic view of an example of a lighting apparatus according to an embodiment of the present disclosure.
Fig. 5B is a schematic view of an example of a moving body with a vehicle lamp according to an embodiment of the present disclosure.
Fig. 6Ais a schematic view of an example of a wearable device according to an embodiment of the present disclosure.
Fig. 6B is a schematic view of another example of a wearable device according to an embodiment of the present disclosure.
Fig. 7A is a schematic view of an example of an image-forming apparatus according to an embodiment of the present disclosure.
Fig. 7B is a schematic view of an example of an exposure light source for an image-forming apparatus according to an embodiment of the present disclosure.
Fig. 7C is a schematic view of an example of an exposure light source for an image-forming apparatus according to an embodiment of the present disclosure.

### DESCRIPTION OF THE EMBODIMENTS

### <<Organic Compound>>

An organic compound according to the present embodiment is an organic compound represented by the following general formula [1]:

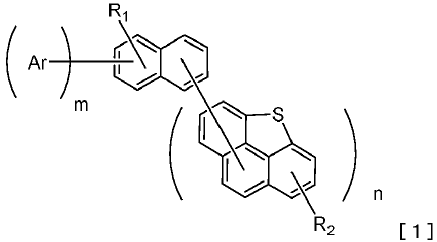

Ar is bonded to one benzene ring of a naphthalene ring, and a phenanthro[4,5-bcd]thiophene ring (hereinafter, sometimes referred to as a "phenanthrothiophene ring") is bonded to the other benzene ring. R₁ is bonded to the naphthalene ring at a position other than the binding position with Ar or the phenanthrothiophene ring. R₂ is bonded to the phenanthrothiophene ring at a position other than the binding position with the naphthalene ring.

When n is 2 or more, the phenanthrothiophene rings may be the same or different. When m is 2 or more, Ars may be the same or different. Each R₁ may be the same or different, and each R₂ may be the same or different.

### <Ar>

In the general formula [1], Ar is selected from the group consisting of a substituted or unsubstituted aryl group and a substituted or unsubstituted heteroaryl group.

Examples of the aryl group include, but are not limited to, aromatic hydrocarbon groups, such as a phenyl group, a naphthyl group, an indenyl group, a biphenyl group, a terphenyl group, a fluorenyl group, a phenanthryl group, a fluoranthenyl group, a triphenylenyl group, a phenanthryl group, a pyrenyl group, a chrysenyl group, and a perylenyl group.

Examples of the heteroaryl group include, but are not limited to, heteroaromatic ring groups, such as a pyridyl group, an oxazolyl group, an oxadiazolyl group, a thiazolyl group, a thiadiazolyl group, a carbazolyl group, an acridinyl group, a phenanthrolyl group, an azaphenanthryl group, a dibenzofuranyl group, a dibenzothiophenyl group, an azatriphenylenyl group, and a diazatriphenylenyl group.

Examples of an additional optional substituent of the aryl group and the heteroaryl group include, but are not limited to, halogen atoms, such as fluorine, chlorine, bromine, and iodine; alkyl groups, such as a methyl group, an ethyl group, a n-propyl group, an isopropyl group, a n-butyl group, and a t-butyl group; alkoxy groups, such as a methoxy group, an ethoxy group, and a propoxy group; amino groups, such as a dimethylamino group, a diethylamino group, a dibenzylamino group, a diphenylamino group, and a ditolylamino group; aryloxy groups, such as a phenoxy group; aromatic hydrocarbon groups, such as a phenyl group and a biphenyl group; heterocyclic groups, such as a pyridyl group and a pyrrolyl group; and a cyano group.

Ar can be a fused ring, a fused ring of three or more 5-membered or 6-membered rings, or a fused ring of four or more 5-membered or 6-membered rings. Ar can be a fused ring other than a phenanthrothiophenyl group.

Ar can be a phenanthryl group, a pyrenyl group, a chrysenyl group, a perylenyl group, a fluoranthenyl group, an azaphenanthryl group, a phenanthrolyl group, or a diazatriphenylenyl group. Ar can be selected from the group consisting of the following [a] to [n] or from the following [a] to [f]. In [a] to [n], * represents a binding position to a naphthalene ring.

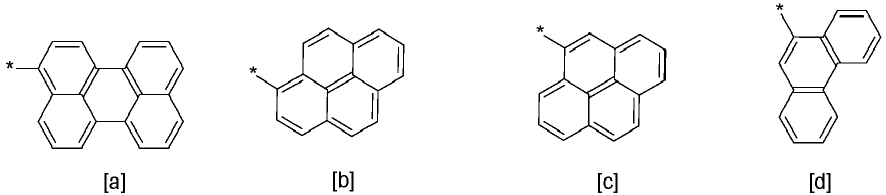

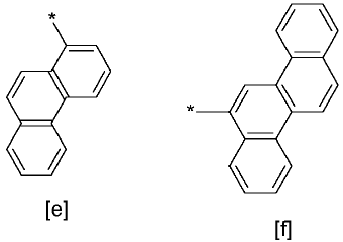

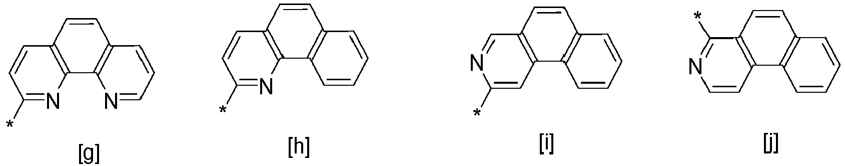

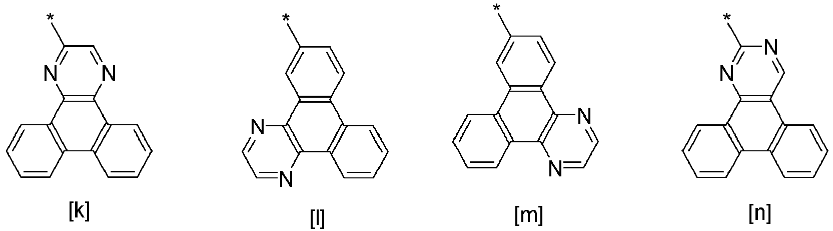

### <R₁ and R₂>

In the general formula [1], R₁ and R₂ are independently selected from the group consisting of a hydrogen atom, a deuterium atom, a halogen atom, a substituted or unsubstituted alkyl group, a substituted or unsubstituted alkoxy group, a substituted or unsubstituted amino group, a substituted or unsubstituted silyl group, a substituted or unsubstituted aryl group, a substituted or unsubstituted heteroaryl group, a substituted or unsubstituted aryloxy group, a substituted or unsubstituted heteroaryloxy group, and a cyano group.

When R₁ denotes an aryl group or a heteroaryl group, the aryl group or the heteroaryl group can be other than a fused ring group.

Examples of the halogen atom include, but are not limited to, fluorine, chlorine, bromine, and iodine.

Examples of the alkyl group include, but are not limited to, a methyl group, an ethyl group, a n-propyl group, an isopropyl group, a n-butyl group, a tert-butyl group, a sec-butyl group, an octyl group, a cyclohexyl group, a 1-adamantyl group, and a 2-adamantyl group. The number of carbon atoms in the alkyl group is preferably 1 or more and 10 or less.

Examples of the alkoxy group include, but are not limited to, a methoxy group, an ethoxy group, a propoxy group, a 2-ethyl-octyloxy group, and a benzyloxy group. The number of carbon atoms in the alkoxy group is preferably 1 or more and 10 or less.

Examples of the amino group include, but are not limited to, an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, an anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, an N,N-dimesitylamino group, an N-phenyl-N-(4-t-butylphenyl)amino group, an N-phenyl-N-(4-trifluoromethylphenyl)amino group, and an N-piperidyl group.

Examples of the silyl group include, but are not limited to, a trimethylsilyl group and a triphenylsilyl group.

Examples of the aryl group include, but are not limited to, a phenyl group, a naphthyl group, an indenyl group, a biphenyl group, a terphenyl group, a fluorenyl group, a phenanthryl group, a fluoranthenyl group, and a triphenylenyl group.

Examples of the heteroaryl group include, but are not limited to, a pyridyl group, an oxazolyl group, an oxadiazolyl group, a thiazolyl group, a thiadiazolyl group, a carbazolyl group, an acridinyl group, a phenanthrolyl group, a dibenzofuranyl group, and a dibenzothiophenyl group.

Examples of the aryloxy group and the heteroaryloxy group include, but are not limited to, a phenoxy group and a thienyloxy group.

Examples of an additional optional substituent of the alkyl group, the alkoxy group, the amino group, the silyl group, the aryl group, the heteroaryl group, the aryloxy group, and the heteroaryloxy group include, but are not limited to, the additional optional substituents of Ar.

### <m, n>

In the general formula [1], m and n are selected from integers of 1 or more and 3 or less.

### <Synthesis Method>

Next, a method for synthesizing the organic compound according to the present embodiment is described. For example, the organic compound according to the present embodiment is synthesized in accordance with the following reaction scheme.

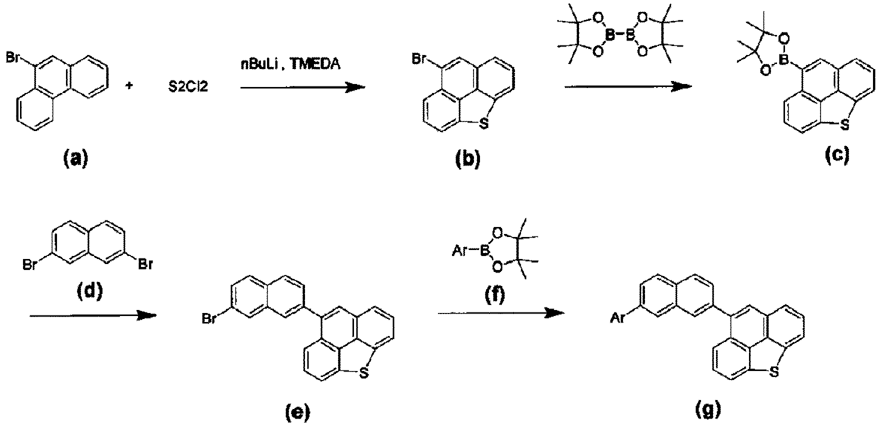

The compounds represented by (a), (d), and (f) can be appropriately chosen to produce various compounds. The synthesis method is not limited to the synthesis scheme described above, and various synthesis schemes and reagents can be used. The synthesis method is described in detail in exemplary embodiments.

### <Properties>

Next, properties of the organic compound according to the present embodiment are described. The organic compound according to the present embodiment has the following characteristics and therefore has good film properties and sublimability. Furthermore, the organic compound can be used to provide an organic light-emitting element with high light emission efficiency and durability.
(1) The naphthalene ring and the Ar group have a large dihedral angle.
(2) The organic compound has a low symmetry structure composed of a phenanthrothiophene ring, a naphthalene ring, and an Ar group.

### (1) The naphthalene ring and the Ar group have a large dihedral angle.

In the disclosure of the organic compound according to the present embodiment, the present inventors have focused on the dihedral angle between a naphthalene ring and an Ar group bonded to the naphthalene ring. More specifically, the organic compound according to the present embodiment has a structure having a third ring, which can be an Ar group as a fused ring, in a structure composed of a phenanthro[4,5-bcd]thiophene ring and a naphthalene ring, and has a dihedral angle with the rings on both sides of the naphthalene ring.

Table 1 shows the results of comparing the dihedral angles of Exemplary Compound A25 and Exemplary Compound A28. Comparative Compound 1-A is Compound 1-A described in Patent Literature 1. The dihedral angles for the bonds were visualized using molecular orbital calculation.

**Table 1**

| | Structure | Dihedral angle of bond "a" |
|---|---|---|
| Exemplary compound A25 | 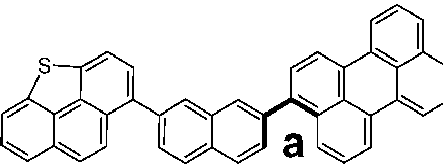 | 56.6° |
| Exemplary compound A28 | 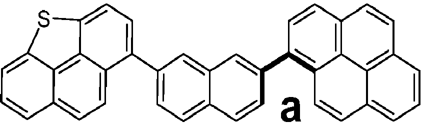 | 55.6° |
| Comparative compound 1-A | 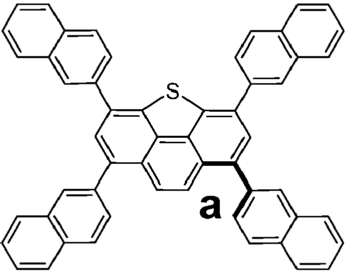 | 52.1° |

Table 1 shows the dihedral angles at the binding position "a" with the largest dihedral angle between the fused ring and the naphthalene ring. Exemplary Compound A25 has a dihedral angle of 56.6 degrees, and Exemplary Compound A28 has a dihedral angle of 55.6 degrees. On the other hand, Comparative Compound 1-A has a dihedral angle of 52.1 degrees. All these structures have a steric hindrance between a peri-hydrogen atom present on the fused ring bonded to the naphthalene ring and a hydrogen atom on the naphthalene ring. In particular, a structure with two tricyclic or higher cyclic fused aromatic rings bonded to the naphthalene ring has a greater planarity inhibiting effect due to steric hindrance. This is because large tricyclic or higher cyclic fused aromatic rings bonded through a relatively small naphthalene ring in the space can maximally exhibit the planarity inhibiting effect. For this reason, it can be seen that Exemplary Compound A25 and Exemplary Compound A28 have a larger dihedral angle than Comparative Compound 1-A. This means that Exemplary Compound A25 and Exemplary Compound A28 have low planarity and form an evaporated film with high amorphousness and higher thermal stability during continuous operation. Thus, a stable amorphous film can be maintained even during the operation of the element, and a long-life organic light-emitting element can be provided.

Thus, the Ar group can be bonded to the naphthalene ring at a substitution position with interference due to steric hindrance between the naphthalene ring and the Ar group. In this case, the Ar group can be a fused ring, a fused ring of three or more 5-membered or 6-membered rings, or a fused ring of four or more 5-membered or 6-membered rings.

### (2) The organic compound has a low symmetry structure composed of a phenanthrothiophene ring, a naphthalene ring, and an Ar group.

The organic compound according to the present embodiment has a low symmetry structure composed of a phenanthrothiophene ring, a naphthalene ring, and an Ar group. For example, when the phenanthrothiophene ring, the naphthalene ring, and the Ar group are each composed of a fused ring with a different structure, a structure with a high molecular weight and low symmetry can be designed. Such a molecular structure has the following effects.

First, the phenanthrothiophene ring, the naphthalene ring, and the Ar group have a conformation that disrupts the planarity, can therefore suppress molecular stacking in which molecules are superposed with each other, make it difficult to crystallize when an evaporated film is formed, and increase amorphousness. High amorphousness, that is, good film properties are suitable for use in organic light-emitting elements because the amorphous state can be easily maintained without crystallization. This is because high amorphousness reduces the generation of crystal grain boundaries, trap levels, and quenchers associated with fine crystallization even during the operation of the element, and high carrier transport ability and efficient emission properties can be maintained. Consequently, an organic light-emitting element with high durability and efficiency can be provided.

Second, a molecular structure with low symmetry suppresses overlapping of molecules, reduces crystallization, and increases amorphousness.

Thus, breaking the symmetry and increasing the molecular weight can increase the amorphousness. Thus, a stable amorphous film can be maintained even during the operation of the element, and a long-life organic light-emitting element can be provided.

When further having the following characteristics, the organic compound according to the present embodiment can be particularly suitable for use in an organic light-emitting element.

### (3) The organic compound has no SP³ carbon.

In the organic compound according to the present embodiment, the phenanthrothiophene ring, the naphthalene ring serving as a linking group, and their optional substituents can have no SP³ carbon, and the Ar group can also have no SP³ carbon.

This is because a carbon-carbon bond of SP³ carbon has low binding energy and is easily cleaved during the operation of the organic light-emitting element. In particular, when used as a host material in a light-emitting layer, the organic compound can be responsible for charge recombination and exciton generation. In this case, the skeleton can be resistant to decomposition because the occurrence of a certain concentration of excitons increases the possibility of being placed in a high-order excitation energy state.

As described below, the stability of binding energy is F1, F2 (C-N) < F4 (SP³ carbon-SP³ carbon) < F3 (SP² carbon-SP² carbon).

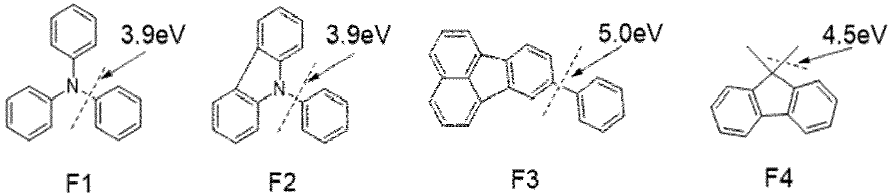

Thus, from the perspective of improving the durability of the element, the organic compound can have no SP³ carbon.

When further having the following characteristics, the organic compound according to the present embodiment has high hole-blocking ability and electron-trapping ability and can be particularly suitable for use in an organic light-emitting element.

### (4) The organic compound has a nitrogen-containing Ar group.

In the disclosure of the organic compound according to the present embodiment, the present inventors have focused on an Ar group bonded to a phenanthrothiophene ring through a naphthalene ring. More specifically, having a nitrogen-containing Ar group, that is, containing a nitrogen atom as an element constituting a ring of the Ar group allows the HOMO level and the LUMO level to be adjusted to be deep (away from the vacuum level). A deep HOMO level and a deep LUMO level can be expected to result in high stability against oxidation and an extended life. A deep HOMO level and a deep LUMO level can also be expected to result in a use for a hole-blocking layer that requires hole-blocking ability. Furthermore, in a light-emitting layer that requires electron-trapping ability, a use as a second host that plays a role of complementing the electron-trapping performance of a host material can be expected.

Table 2 shows the calculated values of the HOMO level and the LUMO level of Exemplary Compound B17, Exemplary Compound B21, and Comparative Compound 1-A.

**Table 2**

| | Structure | HOMO(eV) | LUMO(eV) |
|---|---|---|---|
| Exemplary compound B17 | 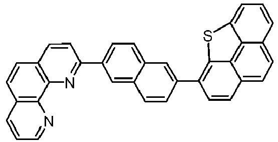 | -5.26 | -1.80 |
| Exemplary compound B21 | 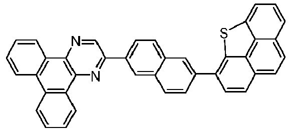 | -5.89 | -1.99 |
| Comparative compound 1-A | 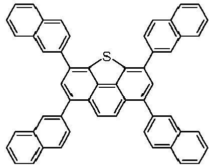 | -5.13 | -1.69 |

As shown in Table 2, Exemplary Compound B17 had a HOMO level of -5.26 eV and a LUMO level of -1.80 eV, and Exemplary Compound B21 had a HOMO level of -5.89 eV and a LUMO level of -1.99 eV. On the other hand, Comparative Compound 1-A had a HOMO level of -5.13 eV and a LUMO level of -1.69 eV. Thus, it can be seen that Exemplary Compound B17 and Exemplary Compound B21 have a deeper value than Comparative Compound 1-A.

Thus, to use this organic compound as a host material for a light-emitting layer or as a carrier-blocking layer in an organic light-emitting element, it is important to have a nitrogen-containing Ar group. As described above, it is possible to provide the element characteristics of high hole-blocking ability, high electron-trapping ability, high efficiency, and long life.

The calculation method in the molecular orbital calculation method utilized a widely used density functional theory (DFT). B3LYP was used as the functional, and 6-31G* was used as the basis function. The molecular orbital calculation method was performed using widely used Gaussian 09 (Gaussian 09, Revision C. 01, M.J. Frisch, G.W. Trucks, H.B. Schlegel, G.E. Scuseria, M.A. Robb, J.R. Cheeseman, G. Scalmani, V. Barone, B. Mennucci, G.A. Petersson, H. Nakatsuji, M. Caricato, X. Li, H.P. Hratchian, A.F. Izmaylov, J. Bloino, G. Zheng, J.L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J.A. Montgomery, Jr., J.E. Peralta, F. Ogliaro, M. Bearpark, J.J. Heyd, E. Brothers, K.N. Kudin, V.N. Staroverov, T. Keith, R. Kobayashi, J. Normand, K. Raghavachari, A. Rendell, J.C. Burant, S.S. Iyengar, J. Tomasi, M. Cossi, N. Rega, J.M. Millam, M. Klene, J.E. Knox, J.B. Cross, V. Bakken, C. Adamo, J. Jaramillo, R. Gomperts, R.E. Stratmann, O. Yazyev, A.J. Austin, R. Cammi, C. Pomelli, J.W. Ochterski, R.L. Martin, K. Morokuma, V.G. Zakrzewski, G.A. Voth, P. Salvador, J.J. Dannenberg, S. Dapprich, A.D. Daniels, O. Farkas, J.B. Foresman, J.V. Ortiz, J. Cioslowski, and D.J. Fox, Gaussian, Inc., Wallingford CT, 2010.).

The organic compound according to the present embodiment has a phenanthrothiophene ring and has the following characteristics. Thus, the organic compound can be particularly suitably for an organic light-emitting element as a host material for a light-emitting layer. (5) The organic compound has a particularly high affinity for a pyrene ring.

To develop an organic compound represented by the general formula [1], the present inventors have focused on a phenanthrothiophene ring as a partial structure of the compound.

As described below, the difference between the phenanthrothiophene ring and a pyrene ring is whether a part of the ring structure is a thiophene ring or a benzene ring. It is known that a heteroatom constituting such a ring structure usually significantly changes the properties. 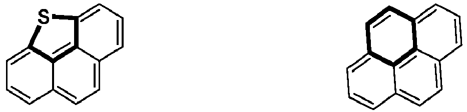 phenanthro[4,5-bcd]thiophene ring pyrene ring

In general, a pyrrole ring with a nitrogen atom, a furan ring with an oxygen atom, and a thiophene ring with a sulfur atom are known as 5-membered heterocyclic compounds. The resonance energy of a 5-membered heterocyclic compound increases in the order of furan < pyrrole < thiophene. Thiophene exhibits the highest aromatic stabilization, which is close to benzene, and furan retains the properties of butadiene. This is because the electronegativities of an oxygen atom and a nitrogen atom are different from the electronegativity of a carbon atom. An oxygen atom or a nitrogen atom with high electronegativity has an influence, induces a dipole moment, and tends to cause a deviation of π electrons. Thus, the resonance stabilization energy of a 5-membered heterocyclic structure is 67.8 kJ/mol in the furan ring and 90.4 kJ/mol in the pyrrole ring, which are lower than 152 kJ/mol in the benzene ring. This indicates a difference in aromatic stabilization. On the other hand, a sulfur atom has the same electronegativity as a carbon atom, induces less dipole moment, has a resonance stabilization energy of 122 kJ/mol, which is close to that of a benzene ring, and provides properties similar to the benzene ring.

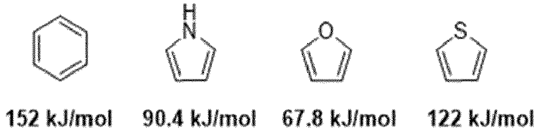

As described above, a thiophene ring with a sulfur atom in the ring structure is similar in property to a benzene ring, and it has been found that a phenanthrothiophene ring and a pyrene ring related to the present disclosure are also similar in property.

For these reasons, it is thought that the phenanthrothiophene ring and the pyrene ring have similar electronic properties and have a high affinity. Thus, the present inventors have found that a stable thin film with high amorphousness and rarely crystallized can be formed by co-depositing a compound with a pyrene ring in the molecule and a compound with a phenanthrothiophene ring as a host material for a light-emitting layer. A compound with a pyrene ring has high compatibility with a dopant material and has sufficient performance as a host material. Even a material with low film stability due to its amorphousness is expected to have enhanced film stability by co-deposition with a compound with a phenanthrothiophene ring. Thus, an organic light-emitting element with high durability without crystallization even in long-term operation can be obtained. When the Ar group is a pyrene ring, the organic compound according to the present embodiment is a compound with the pyrene ring in the molecule and with the phenanthrothiophene ring and is therefore expected to have the same effects as in the case of co-depositing a compound with the pyrene ring in the molecule and a compound with the phenanthrothiophene ring.

As described above, the organic compound according to the present embodiment has low molecular planarity, a broken symmetry, improved amorphousness, and good film properties and sublimability, and is suitable for an organic light-emitting element. Thus, the organic compound according to the present embodiment can be used as a constituent material of an organic light-emitting element to provide an organic light-emitting element with high durability.

### <Specific Examples>

Specific examples of the organic compound according to the present embodiment are described below. However, the present disclosure is not limited to these examples.

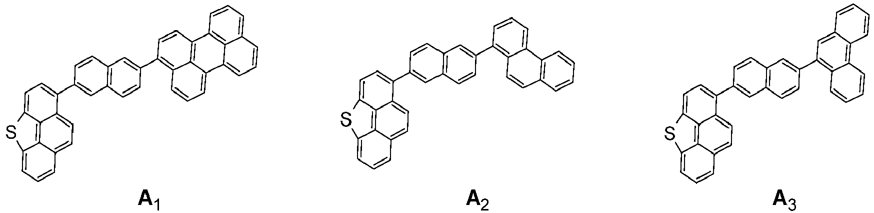

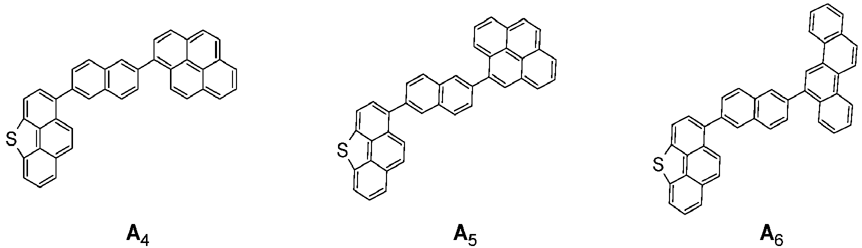

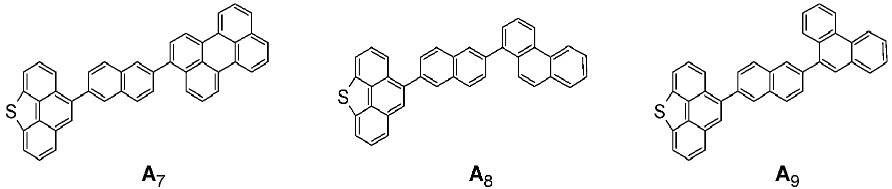

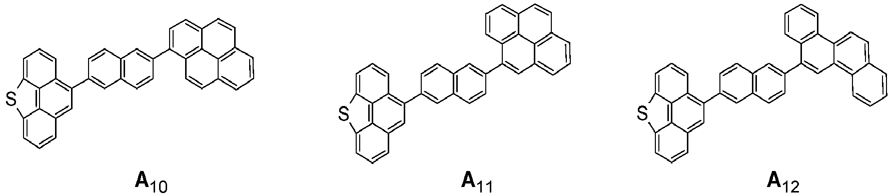

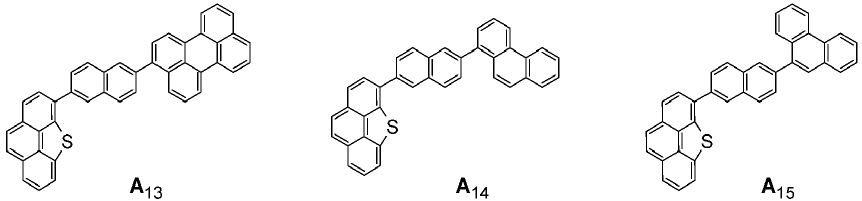

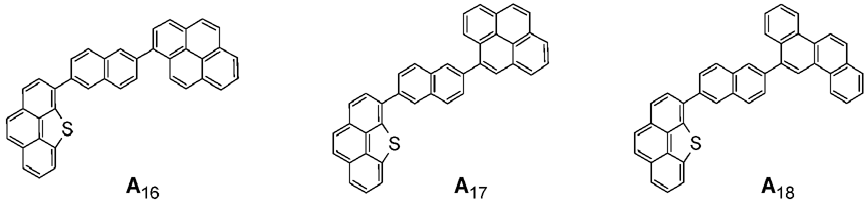

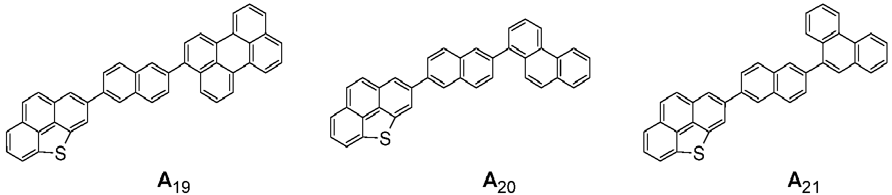

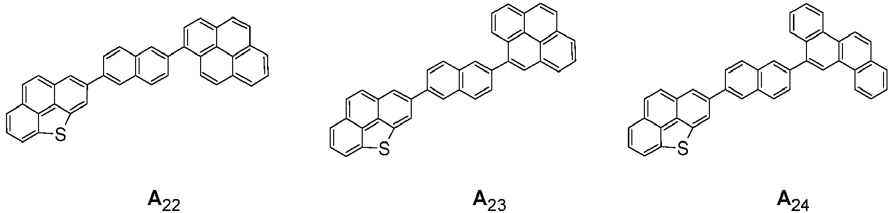

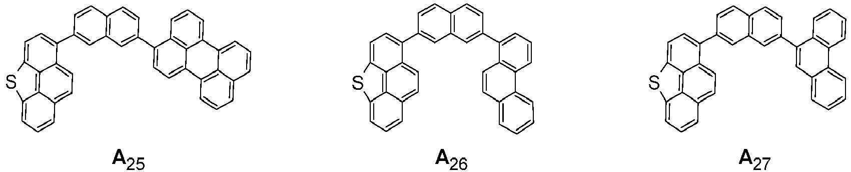

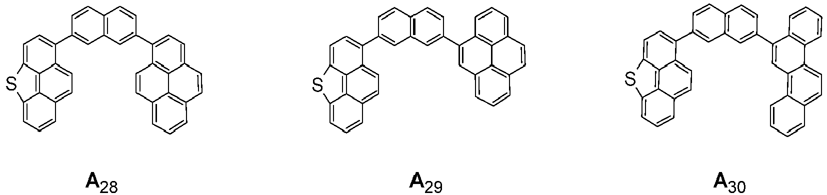

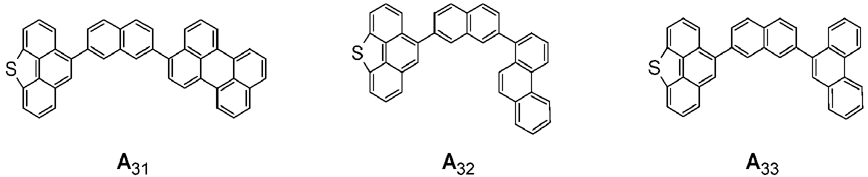

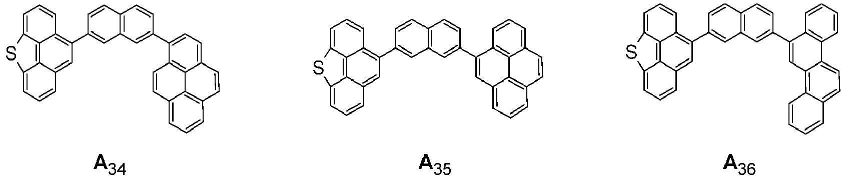

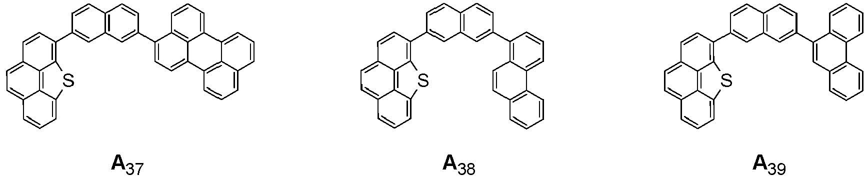

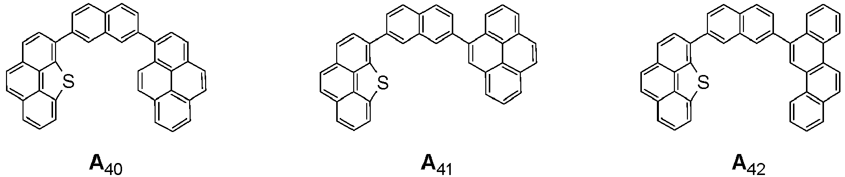

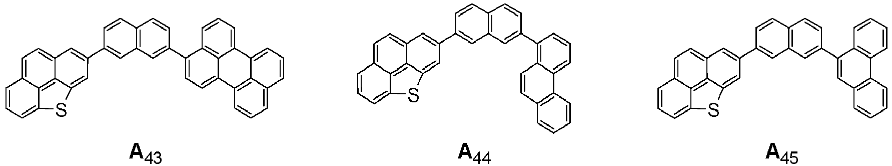

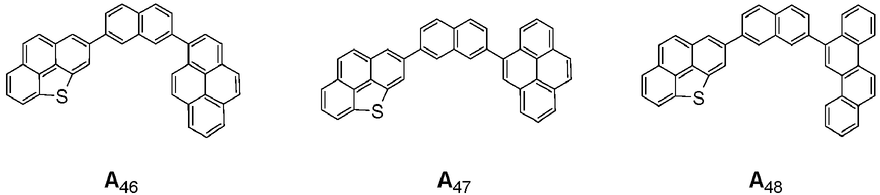

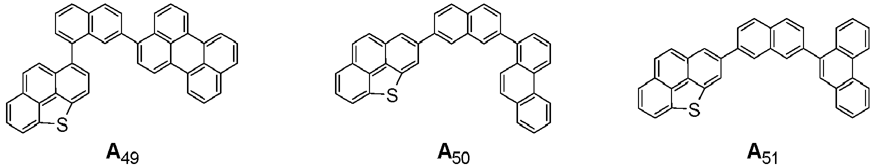

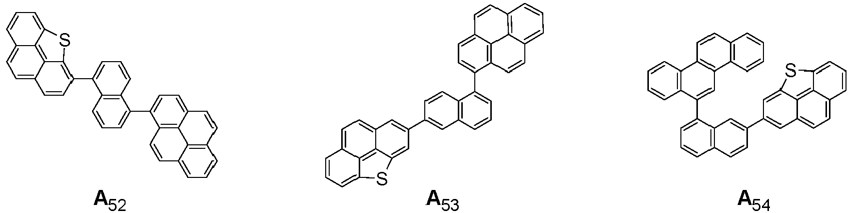

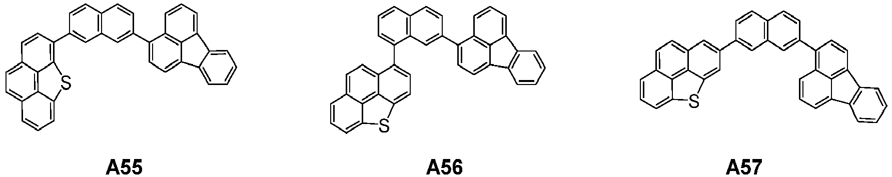

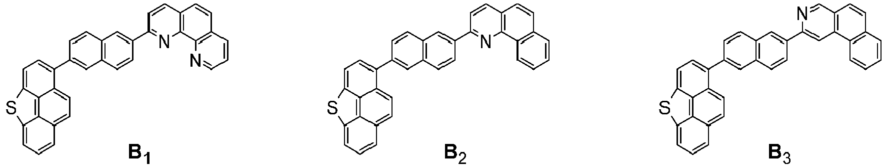

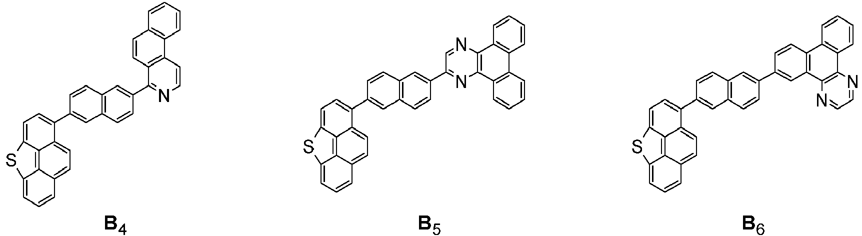

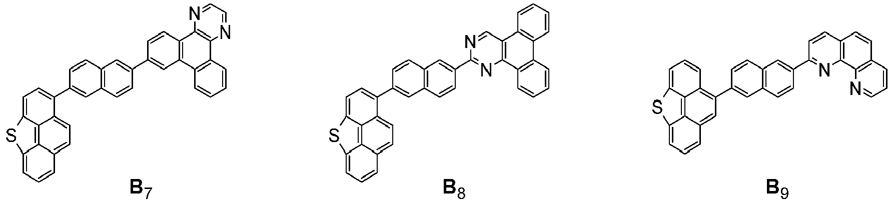

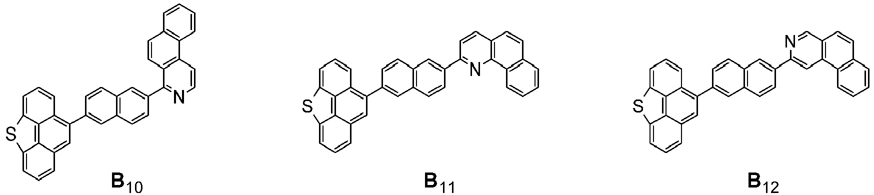

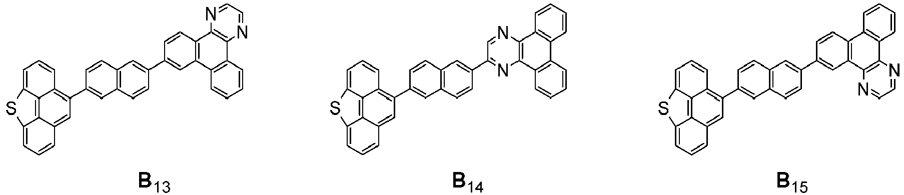

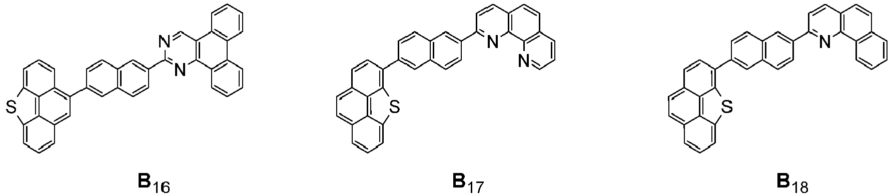

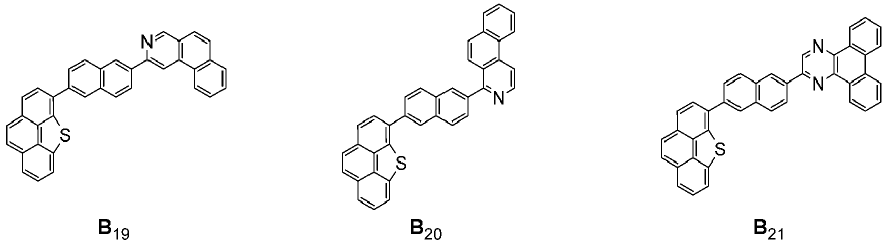

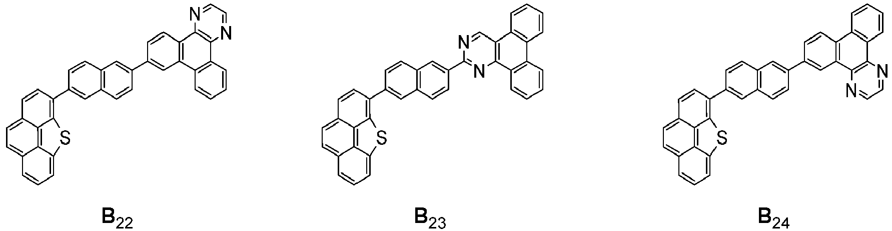

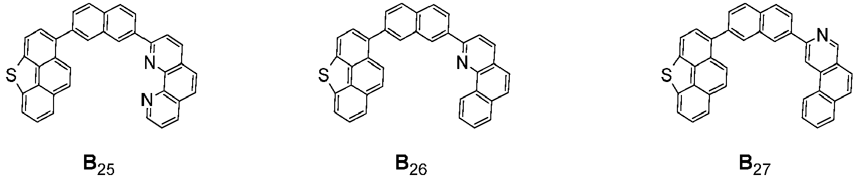

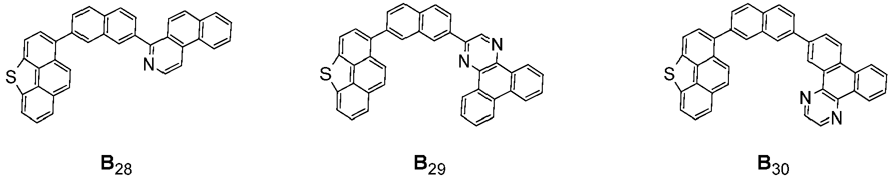

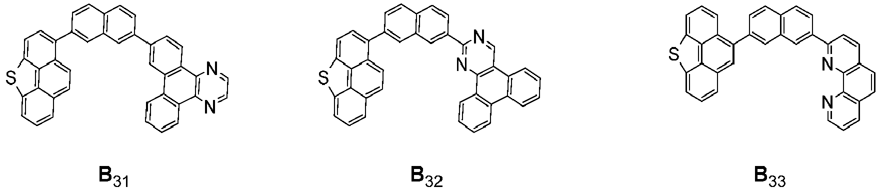

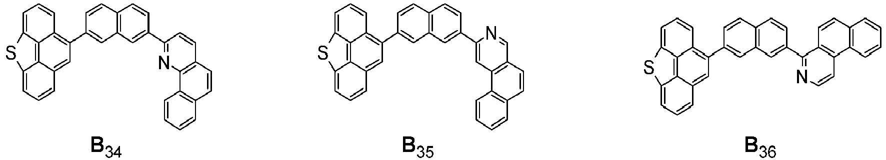

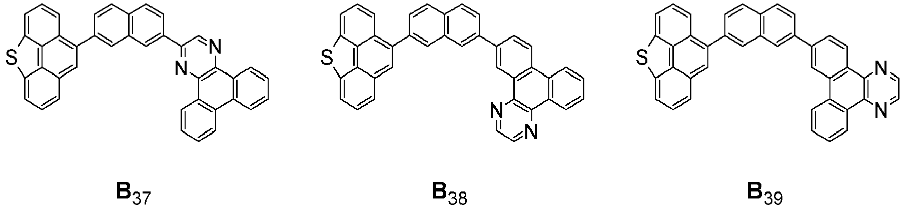

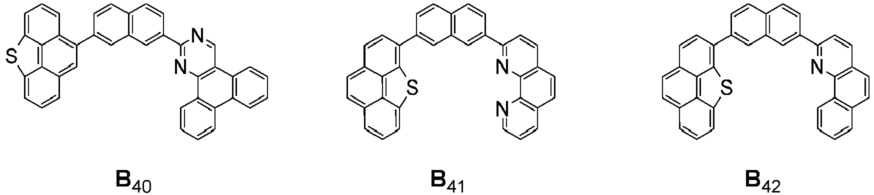

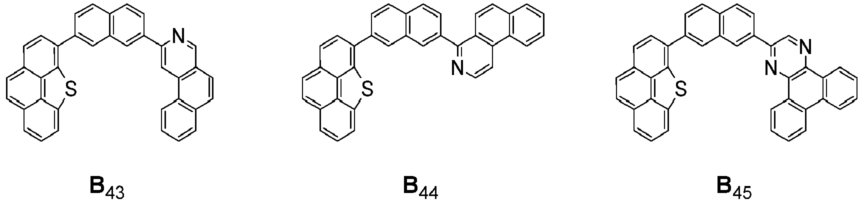

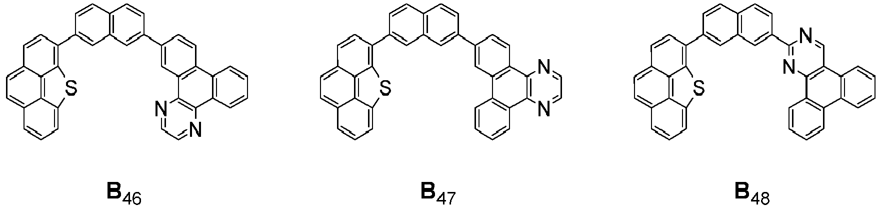

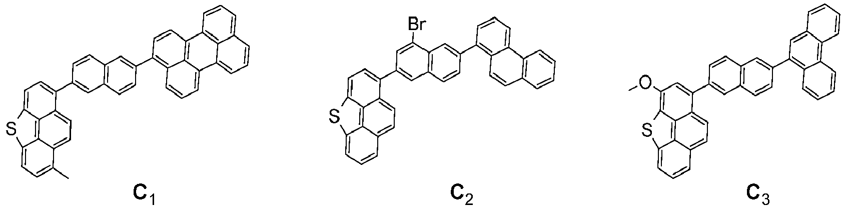

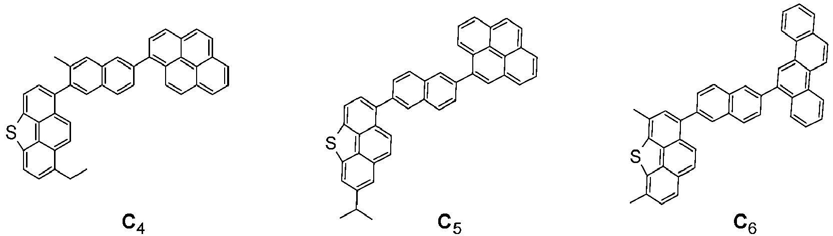

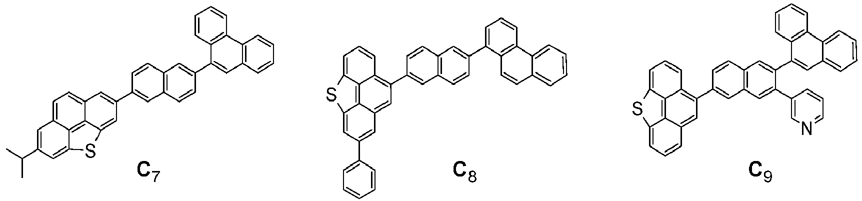

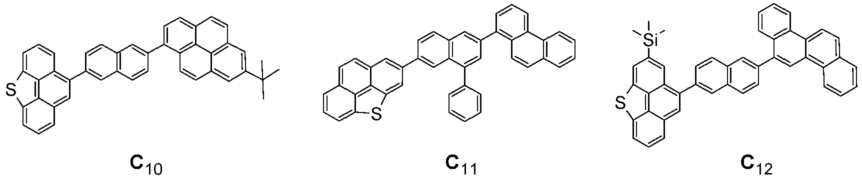

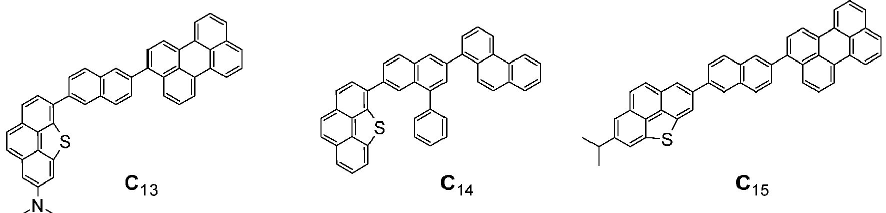

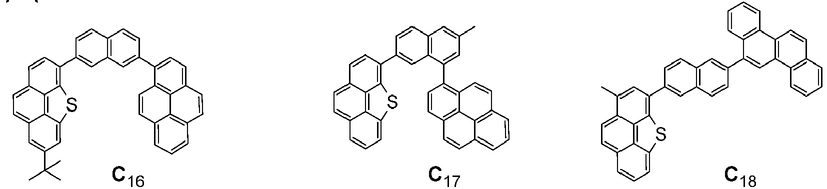

The exemplary compounds belonging to Group A are compounds with a structure in which the Ar group is a fused polycyclic ring composed of a hydrocarbon and has a hydrogen atom at a peri position to the binding position. These compounds have steric hindrance between the naphthalene ring and the Ar group, that is, the fused ring, therefore has a large dihedral angle, has a twisted relationship between the naphthalene ring and the Ar group, and has a structure with low planarity as a whole molecule. This results in a compound that has high amorphousness, has enhanced film stability during long-term continuous operation, and can therefore be expected to have greatly improved operation durability performance.

In the exemplary compounds belonging to Group B, the Ar group is a nitrogen-containing fused polycyclic compound with a nitrogen atom in a part of the ring structure. These compounds have a nitrogen atom in a part of a 6-membered ring structure of the Ar group. Thus, nitrogen with higher electronegativity than carbon attracts π electrons, decreases the electron density of the ring, and has an influence on electronic physical properties as a whole molecule. For example, the HOMO level and the LUMO level become deeper, and the compounds have different properties from the exemplary compounds of Group A. In this case, the compounds with a deeper HOMO level have improved hole-blocking performance and can therefore be used for a hole-blocking layer. Furthermore, the compounds with a deeper LUMO level have enhanced electron injection properties and, when used as a host material, can have improved electron injection properties to a light-emitting layer. A deeper HOMO level can also be expected to improve oxidative stability. The compounds with these characteristics can be expected to significantly improve the operation durability performance by eliminating charge deviation in a light-emitting layer during long-term continuous operation.

The exemplary compounds belonging to Group C are compounds with a group other than a hydrogen atom as R₁ or R₂ or with a substituent on the phenanthrothiophene ring. In these compounds, R₁ or R₂ other than a hydrogen atom or the substituent on the phenanthrothiophene ring plays a role as a steric hindrance group and suppresses intermolecular stacking. Thus, the compounds have high amorphousness in a thin film state after film formation, have less intermolecular rearrangement due to the effects of the steric hindrance group, and have very high sustainability of an amorphous film. Furthermore, the compounds have less intermolecular stacking, play a role of reducing localization of dopant molecules when used as a host material for a light-emitting layer, and are expected to have an effect of being easily and uniformly incorporated into a film.

### <Conditions in Light-Emitting Layer>

Furthermore, the compound according to the present embodiment can be used in a light-emitting layer in an organic light-emitting element under the following conditions.
(6) The compound according to the present embodiment constitutes 0.5% by mass or more and 99% by mass or less in a light-emitting layer.
(7) The compound according to the present embodiment is used as a first host material in a light-emitting layer, and a second host material to be mixed as a second component is a compound with two or more fused rings of either a phenanthrene ring or a pyrene ring at least in the molecular structure.

These conditions are described below.
(6) The compound according to the present embodiment constitutes 0.5% by mass or more and 99% by mass or less in a light-emitting layer.

When the organic compound according to the present embodiment is used in a light-emitting layer, the organic compound content is preferably 0.5% by mass or more and 99% by mass or less. Due to its enhanced amorphousness, the organic compound according to the present embodiment is a material suitable for a host material for a light-emitting layer and can constitute 50% by mass or more. Even when constituting 99% by mass, the organic compound is rarely crystallized and exhibits a function with good characteristics.

From the perspective of improving the film properties of a light-emitting layer, the organic compound may also be used as an assist material. 0.5% by mass or more and 50% by mass or less of the organic compound may be used as an assist material.

This is due to the structural characteristics of the organic compound according to the present embodiment. It is possible to provide an organic light-emitting element with good characteristics in which the compound is less likely to aggregate and crystal grain boundaries associated with molecular aggregation are rarely formed even while the organic light-emitting element is driven. (7) The compound according to the present embodiment is used as a first host material in a light-emitting layer, and a second host material to be mixed as a second component is a compound with two or more fused rings of either a phenanthrene ring or a pyrene ring at least in the molecular structure.

A light-emitting layer may further contain a second component. More specifically, the organic compound according to the present embodiment may be used as a first host material in a light-emitting layer, and a second host material may be contained as a second component. The second component can have highest occupied molecular orbital energy (HOMO energy) higher (shallower) than that of the organic compound according to the present embodiment. The second component can be a compound with a fused ring of either a phenanthrene ring or a pyrene ring or with two or more pyrene rings in the molecule. The organic compound according to the present embodiment, which has low symmetry and high amorphousness, can be co-deposited with a material having two or more fused rings of either a phenanthrene ring or a pyrene ring and having high symmetry and low amorphousness of a film and can be used as a double host material. In such a case, 0.5% by mass or more of the compound according to the present embodiment can be expected to be effective. Even 0.5% by mass of the compound in the film can improve the amorphousness of the second component with high symmetry. The second component content with respect to the total of the organic compound according to the present embodiment and the second component is preferably 10% by mass or less.

### <<Organic Light-Emitting Element>>

An organic light-emitting element according to the present embodiment includes a pair of electrodes (a first electrode and a second electrode) and an organic compound layer between the pair of electrodes. When the organic compound layer is a laminate of a plurality of layers, the organic compound layer may have a hole-injection layer, a hole-transport layer, an electron-blocking layer, a hole/exciton-blocking layer, an electron-transport layer, and/or an electron-injection layer, in addition to a light-emitting layer. The light-emitting layer may be a single layer or a laminate of a plurality of layers. In the presence of a plurality of light-emitting layers, a charge generation layer may be disposed between light-emitting layers. The charge generation layer can be composed of a compound with a low (deep) LUMO level, more specifically a compound with a LUMO level lower than that of a hole-transport layer. The charge generation layer can be composed of a compound with a LUMO level lower than the HOMO level of a hole-transport layer.

In the organic light-emitting element according to the present embodiment, at least one organic compound layer or a light-emitting layer contains the organic compound according to the present embodiment. The organic compound according to the present embodiment can be contained in a light-emitting layer as a host, an assist, or a guest, or as a host or an assist.

The host is a compound with the highest mass ratio among the compounds constituting a light-emitting layer. The guest is a compound that has a lower mass ratio than the host among the compounds constituting a light-emitting layer and that is a principal light-emitting compound. The concentration of the host in a light-emitting layer is preferably 10% by mass or more and 99% by mass or less, more preferably 10% by mass or more and 90% by mass or less, still more preferably 20% by mass or more and 80% by mass or less, still more preferably 30% by mass or more and 70% by mass or less, of the entire light-emitting layer. The concentration of the assist is preferably 0.5% by mass or more and 50% by mass or less.

The concentration of the guest is 0.01% by mass or more and 50% by mass or less, preferably 0.1% by mass or more and 20% by mass or less, of the total amount of the constituent materials of a light-emitting layer. From the perspective of reducing concentration quenching, the concentration of the guest is particularly preferably 10% by mass or less. The guest may be uniformly contained or may have a concentration gradient throughout a layer in which the host serves as a matrix. Alternatively, the guest may be partially contained in a specific region in the layer, and a light-emitting layer may have a region containing only the host and no guest.

A light-emitting layer may contain a light-emitting material with lowest unoccupied molecular orbital energy lower (deeper) than the lowest unoccupied molecular orbital energy (LUMO energy) of the organic compound according to the present embodiment. Such a light-emitting material may have a 5-membered ring in its molecular structure. A light-emitting layer may contain a light-emitting material with highest occupied molecular orbital energy (HOMO energy) higher (shallower) than the highest occupied molecular orbital energy of the organic compound according to the present embodiment.

A light-emitting layer may be of monolayer or multilayer or may have a mixture of colors by containing a light-emitting material of another emission color. The term "multilayer", as used herein, refers to a laminate of a light-emitting layer and another light-emitting layer. In such a case, the organic light-emitting element may have any emission color. More specifically, the emission color may be white or a neutral color. For a white emission color, for example, when a light-emitting layer has a blue emission color, another light-emitting layer has an emission color different from blue, that is, green or red.

Furthermore, a third light-emitting layer that emits blue light and a charge generation layer may be provided between a light-emitting layer or a laminated light-emitting layer and the first or second electrode. The charge generation layer has a function as a tandem element, and an electron generated from the charge generation layer and a hole injected from one electrode are recombined and generate an exciton, and a hole generated from the charge generation layer and an electron injected from the other electrode are recombined and generate an exciton. The charge generation layer may contain a compound with lowest unoccupied molecular orbital energy (LUMO) lower than the highest occupied molecular orbital energy (HOMO) of a light-emitting layer. Thus, a white-light-emitting element can be provided by forming a tandem element structure using a laminated light-emitting layer including a light-emitting layer according to the present embodiment. The third light-emitting layer contains at least a third organic compound and a fourth organic compound. The third organic compound is a host material, and the fourth organic compound is a blue-light-emitting material.

A specific element structure of the organic light-emitting element according to the present embodiment may be a multilayer element structure including an electrode layer and an organic compound layer shown in the following (a) to (f) sequentially stacked on a substrate. In any of the element structures, the organic compound layer always includes a light-emitting layer containing a light-emitting material.
(a) positive electrode/light-emitting layer/negative electrode
(b) positive electrode/hole-transport layer/light-emitting layer/electron-transport layer/negative electrode
(c) positive electrode/hole-transport layer/light-emitting layer/electron-transport layer/electron-injection layer/negative electrode
(d) positive electrode/hole-injection layer/hole-transport layer/light-emitting layer/electron-transport layer/negative electrode
(e) positive electrode/hole-injection layer/hole-transport layer/light-emitting layer/electron-transport layer/electron-injection layer/negative electrode
(f) positive electrode/hole-transport layer/electron-blocking layer/light-emitting layer/hole-blocking layer/electron-transport layer/negative electrode

However, these element structure examples are only very basic element structures, and the present disclosure is not limited to these structures. Various layer structures are possible; for example, an insulating layer, an adhesive layer, or an interference layer is formed at an interface between an electrode and an organic compound layer, an electron-transport layer or a hole-transport layer is composed of two layers with different ionization potentials, or a light-emitting layer is composed of two layers formed of different light-emitting materials.

Among the element structures shown in (a) to (f), the structure (f) has both an electron-blocking layer and a hole-blocking layer. Thus, the electron-blocking layer and the hole-blocking layer in (f) can securely confine both carriers of holes and electrons in the light-emitting layer. Thus, the organic light-emitting element has no carrier leakage and high light emission efficiency.

The mode (element form) of extracting light from a light-emitting layer may be a bottom emission mode of extracting light from an electrode on the substrate side or a top emission mode of extracting light from the side opposite to the substrate side. The mode may also be a double-sided extraction mode of extracting light from the substrate side and from the side opposite to the substrate side.

The organic compound according to the present embodiment can be used as a constituent material of an organic compound layer other than the light-emitting layer constituting the organic light-emitting element according to the present embodiment. More specifically, the organic compound according to the present embodiment may be used as a constituent material of an electron-transport layer, an electron-injection layer, a hole-transport layer, a hole-injection layer, and/or a hole-blocking layer. In such a case, the organic light-emitting element may have any emission color. More specifically, the emission color may be white or a neutral color.

### <Other Compounds>

If necessary, the organic light-emitting element according to the present embodiment may also include a known low-molecular-weight or high-molecular-weight hole-injection compound or hole-transport compound, host compound, light-emitting compound, electron-injection compound, or electron-transport compound. Examples of these compounds are described below.

The hole-injection/transport material can be a material with high hole mobility to facilitate the injection of holes from a positive electrode and to transport the injected holes to a light-emitting layer. Furthermore, a material with a high glass transition temperature can be used to reduce degradation of film quality, such as crystallization, in an organic light-emitting element. Examples of the low-molecular-weight or high-molecular-weight material with hole-injection/transport ability include, but are not limited to, triarylamine derivatives, aryl carbazole derivatives, phenylenediamine derivatives, stilbene derivatives, phthalocyanine derivatives, porphyrin derivatives, polyvinylcarbazole, polythiophene, and other electrically conductive polymers. The hole-injection/transport material can also be used for an electron-blocking layer. Specific examples of compounds that can be used as hole-injection/transport materials include, but are not limited to, the following.

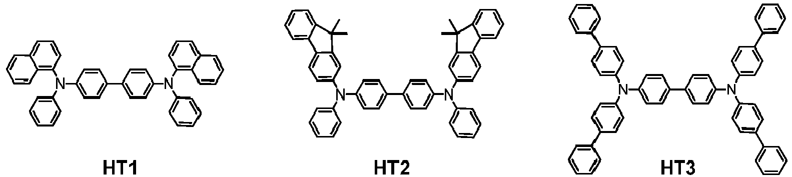

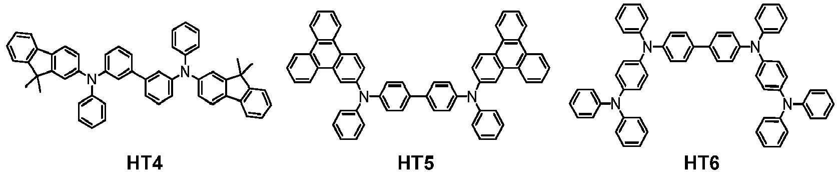

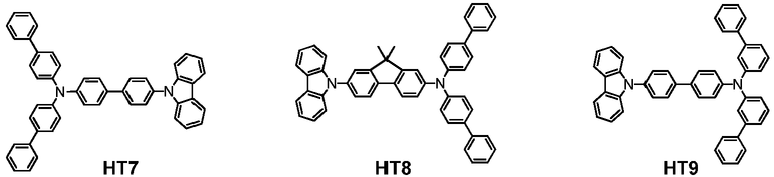

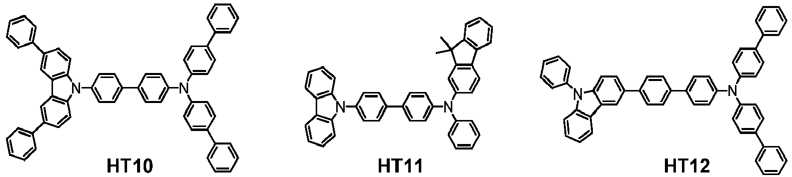

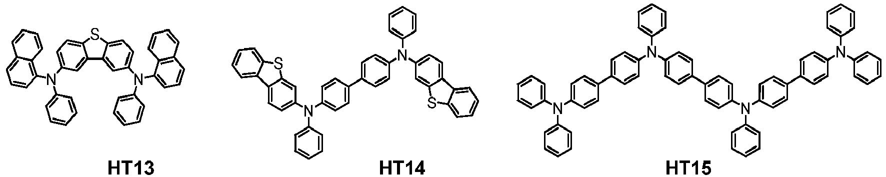

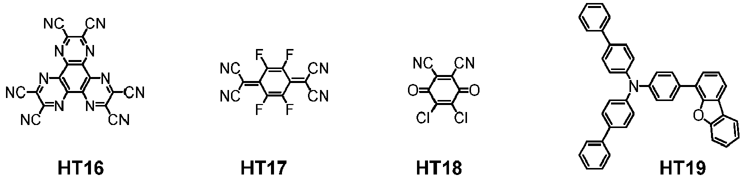

Examples of a light-emitting material mainly related to the light-emitting function include fused-ring compounds (for example, fluorene derivatives, naphthalene derivatives, pyrene derivatives, perylene derivatives, tetracene derivatives, anthracene derivatives, rubrene, or the like), quinacridone derivatives, coumarin derivatives, stilbene derivatives, organoaluminum complexes, such as tris(8-quinolinolato)aluminum, iridium complexes, platinum complexes, rhenium complexes, copper complexes, europium complexes, ruthenium complexes, and polymer derivatives, such as poly(phenylene vinylene) derivatives, polyfluorene derivatives, and polyphenylene derivatives. Specific examples of compounds that can be used as light-emitting materials include, but are not limited to, the following.

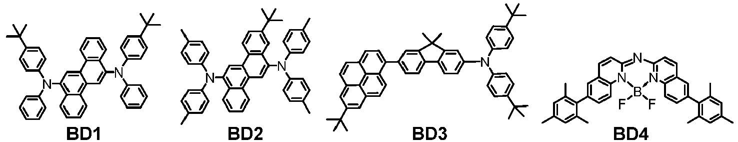

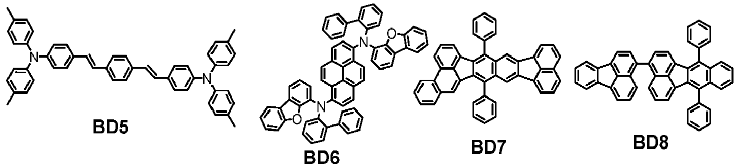

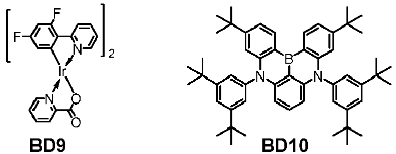

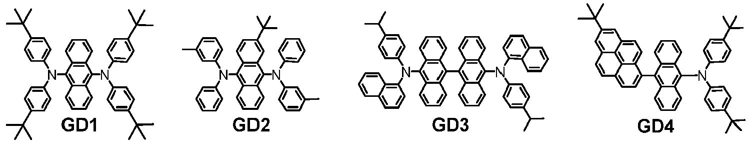

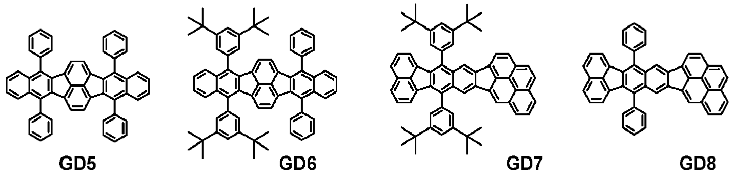

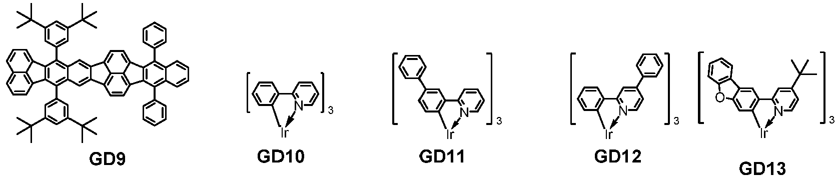

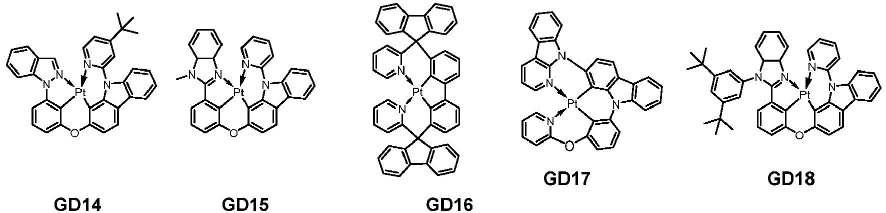

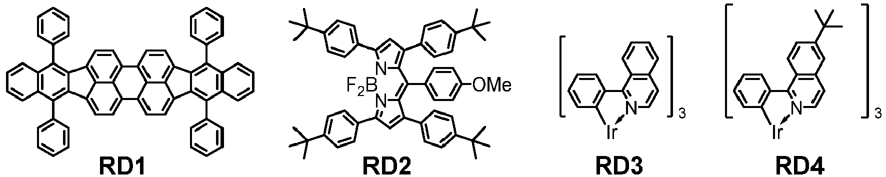

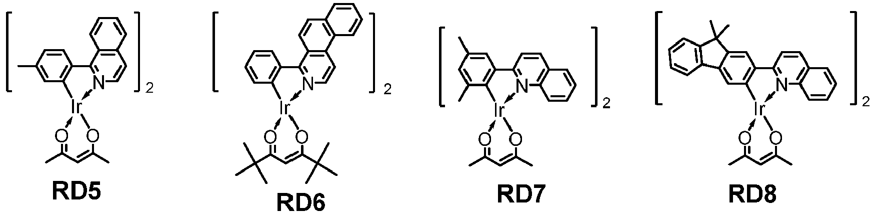

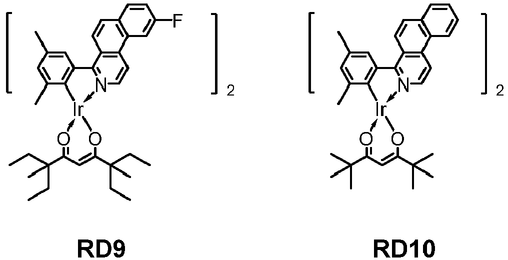

A compound other than the organic compound according to the present embodiment may be contained as a second component as a light-emitting layer host or a light-emitting assist material contained in a light-emitting layer. Examples of the second component include aromatic hydrocarbon compounds and derivatives thereof, carbazole derivatives, azine derivatives, xanthone derivatives, dibenzofuran derivatives, dibenzothiophene derivatives, organoaluminum complexes, such as tris(8-quinolinolato) aluminum, and organoberyllium complexes.

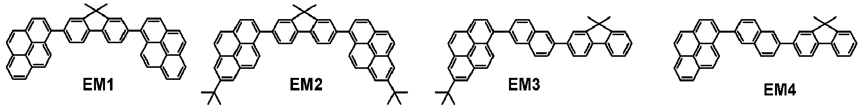

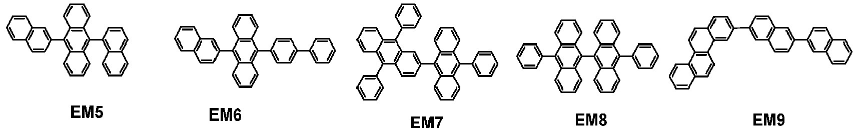

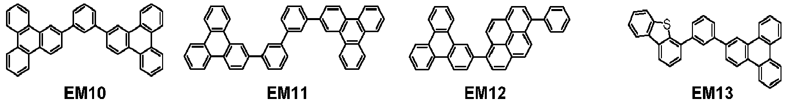

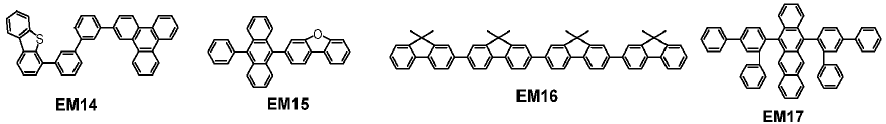

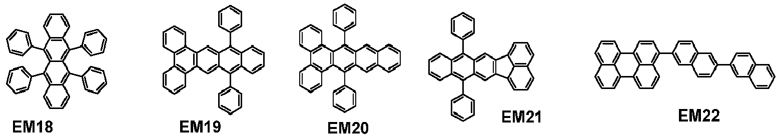

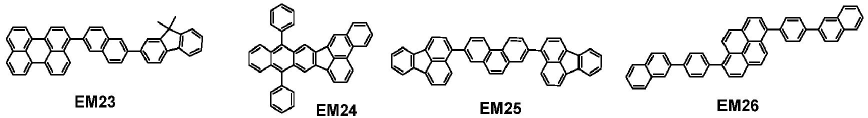

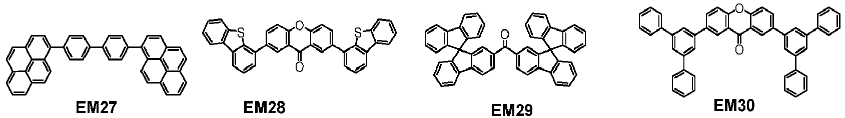

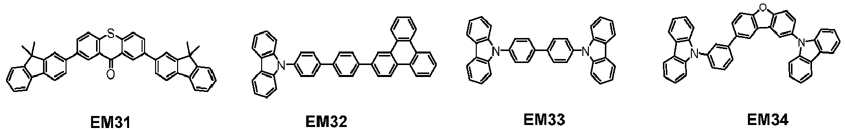

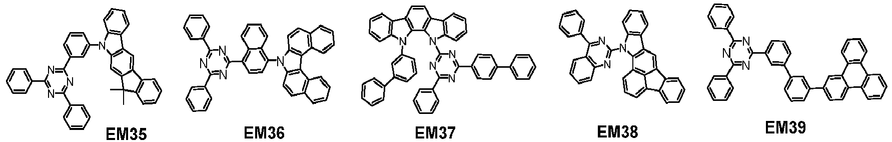

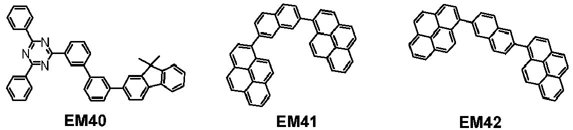

An electron-transport material can be selected from materials that can transport electrons injected from the negative electrode to the light-emitting layer and is selected in consideration of the balance with the hole mobility of a hole-transport material and the like. Examples of materials with electron-transport ability include, but are not limited to, oxadiazole derivatives, oxazole derivatives, pyrazine derivatives, triazole derivatives, triazine derivatives, quinoline derivatives, quinoxaline derivatives, phenanthroline derivatives, organoaluminum complexes, and fused-ring compounds (for example, fluorene derivatives, naphthalene derivatives, chrysene derivatives, and anthracene derivatives). Furthermore, the electron-transport material is also suitable for use in a hole-blocking layer. Specific examples of compounds that can be used as electron-transport materials include, but are not limited to, the following.

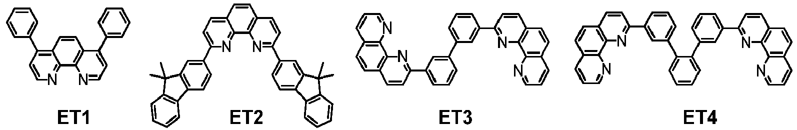

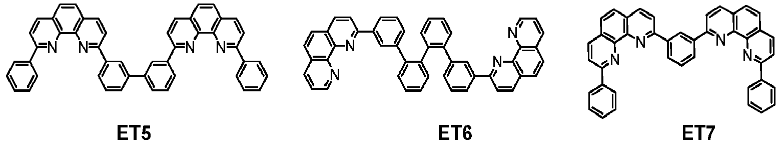

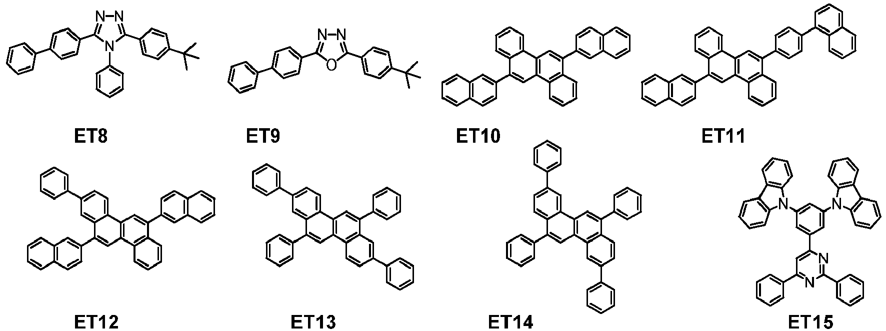

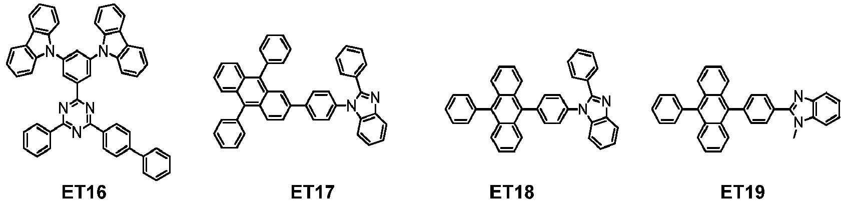

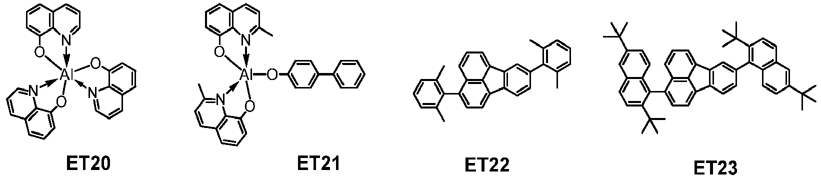

The electron-injection material can be selected from materials that can easily inject electrons from the negative electrode and is selected in consideration of the balance with the hole injection properties and the like. The organic compound may be an n-type dopant or a reducing dopant. Examples include compounds containing an alkali metal, such as lithium fluoride, lithium complexes, such as lithium quinolinol, benzimidazolidene derivatives, imidazolidene derivatives, fulvalene derivatives, and acridine derivatives. It can also be used in combination with the electron-transport material.

### <Structure of Organic Light-Emitting Element>

An organic light-emitting element includes an insulating layer, a first electrode, an organic compound layer, and a second electrode on a substrate. A protective layer, a color filter, a microlens, or the like may be provided on the second electrode. When a color filter is provided, a planarization layer may be provided between the color filter and a protective layer. The planarization layer may be composed of an acrylic resin or the like. The same applies to a planarization layer provided between a color filter and a microlens.

### [Substrate]

The substrate may be formed of quartz, glass, a silicon wafer, resin, metal, or the like. The substrate may have a switching element, such as a transistor, and wiring, on which an insulating layer may be provided. The insulating layer may be composed of any material, provided that the insulating layer can have a contact hole for wiring between the insulating layer and the first electrode and is insulated from unconnected wires. For example, the insulating layer may be formed of a resin, such as polyimide, silicon oxide, or silicon nitride.

### [Electrodes]

A pair of electrodes can be used as electrodes. The pair of electrodes may be a positive electrode and a negative electrode. When an electric field is applied in a direction in which the organic light-emitting element emits light, an electrode with a high electric potential is a positive electrode, and the other electrode is a negative electrode. In other words, the electrode that supplies holes to the light-emitting layer is a positive electrode, and the electrode that supplies electrons to the light-emitting layer is a negative electrode.

A constituent material of the positive electrode can have as large a work function as possible. Examples of the constituent material include metal elements, such as gold, platinum, silver, copper, nickel, palladium, cobalt, selenium, vanadium, and tungsten, mixtures thereof, alloys thereof, and metal oxides, such as tin oxide, zinc oxide, indium oxide, indium tin oxide (ITO), and indium zinc oxide. Electrically conductive polymers, such as polyaniline, polypyrrole, and polythiophene, may also be used.

These electrode materials may be used alone or in combination. The positive electrode may be composed of a single layer or a plurality of layers.

When used as a reflective electrode, for example, chromium, aluminum, silver, titanium, tungsten, molybdenum, an alloy thereof, or a laminate thereof can be used. These materials can also function as a reflective film that does not have a role as an electrode. When used as a transparent electrode, an oxide transparent conductive layer, such as indium tin oxide (ITO) or indium zinc oxide, can be used. However, the present disclosure is not limited thereto. The electrodes may be formed by photolithography.

A constituent material of the negative electrode can be a material with a small work function. For example, an alkali metal, such as lithium, an alkaline-earth metal, such as calcium, a metal element, such as aluminum, titanium, manganese, silver, lead, or chromium, or a mixture thereof may be used. An alloy of these metal elements may also be used. For example, magnesium-silver, aluminum-lithium, aluminum-magnesium, silver-copper, or zinc-silver may be used. A metal oxide, such as indium tin oxide (ITO), may also be used. These electrode materials may be used alone or in combination. The negative electrode may be composed of a single layer or a plurality of layers. In particular, silver can be used, and a silver alloy can be used to reduce the aggregation of silver. As long as the aggregation of silver can be reduced, the alloy may have any ratio. For example, the ratio of silver to another metal may be 1:1, 3:1, or the like.

The negative electrode may be, but is not limited to, an oxide conductive layer, such as ITO, for a top emission element or a reflective electrode, such as aluminum (Al), for a bottom emission element. The negative electrode may be formed by any method. A direct-current or alternating-current sputtering method can achieve good film coverage and easily decrease resistance.

### [Organic Compound Layer]

The organic compound layer may be formed of a single layer or a plurality of layers. Depending on their functions, the plurality of layers may be referred to as a hole-injection layer, a hole-transport layer, an electron-blocking layer, a light-emitting layer, a hole-blocking layer, an electron-transport layer, or an electron-injection layer. A plurality of light-emitting layers may be provided, and a charge generation layer may be provided between the plurality of light-emitting layers. The organic compound layer is composed mainly of an organic compound and may contain an inorganic atom or an inorganic compound. For example, copper, lithium, magnesium, aluminum, iridium, platinum, molybdenum, zinc, or the like may be contained. The organic compound layer may be located between the first electrode and the second electrode and may be in contact with the first electrode and the second electrode.

An organic compound layer (a hole-injection layer, a hole-transport layer, an electron-blocking layer, a light-emitting layer, a hole-blocking layer, an electron-transport layer, an electron-injection layer, a charge generation layer, or the like) constituting an organic light-emitting element according to an embodiment of the present disclosure is formed by the following method.

An organic compound layer constituting an organic light-emitting element according to an embodiment of the present disclosure can be formed by a dry process, such as a vacuum evaporation method, an ionized deposition method, sputtering, or plasma. Instead of the dry process, a wet process may also be employed in which a layer is formed by a known coating method (for example, spin coating, dipping, a casting method, an LB method, an ink jet method, or the like) using an appropriate solvent.

A layer formed by a vacuum evaporation method, a solution coating method, or the like undergoes little crystallization or the like and has high temporal stability. When a film is formed by a coating method, the film may also be formed in combination with an appropriate binder resin.

Examples of the binder resin include, but are not limited to, polyvinylcarbazole resins, polycarbonate resins, polyester resins, ABS resins, acrylic resins, polyimide resins, phenolic resins, epoxy resins, silicone resins, and urea resins.

These binder resins may be used alone or in combination as a homopolymer or a copolymer. If necessary, an additive agent, such as a known plasticizer, oxidation inhibitor, and/or ultraviolet absorbent, may also be used.

### [Protective Layer]

A protective layer may be provided on the second electrode. For example, a glass sheet with a moisture absorbent may be attached to the second electrode to decrease the amount of water or the like entering the organic compound layer and to reduce the occurrence of display defects. In another embodiment, a passivation film of silicon nitride or the like may be provided on the second electrode to decrease the amount of water or the like entering the organic compound layer. For example, the second electrode may be formed and then transferred to another chamber without breaking the vacuum, and a silicon nitride film with a thickness of 2 µm may be formed as a protective layer by a chemical vapor deposition (CVD) method. The film formation by the CVD method may be followed by the formation of a protective layer by an atomic layer deposition (ALD) method. A film formed by the ALD method may be formed of any material such as silicon nitride, silicon oxide, or aluminum oxide. Silicon nitride may be further formed by the CVD method on the film formed by the ALD method. The film formed by the ALD method may have a smaller thickness than the film formed by the CVD method. More specifically, the thickness may be 50% or less or even 10% or less.

### [Color Filter]

A color filter may be provided on the protective layer. For example, a color filter that matches the size of the organic light-emitting element may be provided on another substrate and may be bonded to the substrate on which the organic light-emitting element is provided, or a color filter may be patterned on the protective layer by photolithography. The color filter may be composed of a polymer.

### [Planarization Layer]

A planarization layer may be provided between the color filter and the protective layer. The planarization layer is provided to reduce the roughness of the underlayer. The planarization layer is sometimes referred to as a material resin layer with any purpose. The planarization layer may be composed of an organic compound and can be composed of a high-molecular-weight compound, though it may be composed of a low-molecular-weight compound.

The planarization layer may be provided above and below the color filter, and the constituent materials thereof may be the same or different. Specific examples include polyvinylcarbazole resins, polycarbonate resins, polyester resins, ABS resins, acrylic resins, polyimide resins, phenolic resins, epoxy resins, silicone resins, and urea resins.

### [Microlens]

An organic light-emitting element or an organic light-emitting apparatus may include an optical member, such as a microlens, on the light output side. The microlens may be composed of an acrylic resin, an epoxy resin, or the like. The microlens may be used to increase the amount of light extracted from the organic light-emitting element or the organic light-emitting apparatus and control the direction of the extracted light. The microlens may have a hemispherical shape. For a hemispherical microlens, the vertex of the microlens is a contact point between the hemisphere and a tangent line parallel to the insulating layer among the tangent lines in contact with the hemisphere. The vertex of the microlens in a cross-sectional view can be determined in the same manner. More specifically, the vertex of the microlens in a cross-sectional view is a contact point between the semicircle of the microlens and a tangent line parallel to the insulating layer among the tangent lines in contact with the semicircle.

The midpoint of the microlens can also be defined. In a cross section of the microlens, a midpoint of a line segment from one end point to the other end point of the arc can be referred to as a midpoint of the microlens. A cross section in which the vertex and the midpoint are determined may be perpendicular to the insulating layer.

### [Opposite Substrate]

An opposite substrate may be provided on the planarization layer. The opposite substrate is so called because it faces the substrate. The opposite substrate may be composed of the same material as the substrate. When the substrate is a first substrate, the opposite substrate may be a second substrate.

### [Pixel Circuit]

An organic light-emitting apparatus including an organic light-emitting element may include a pixel circuit coupled to the organic light-emitting element. The pixel circuit may be of an active matrix type, which independently controls the light emission of a first light-emitting element and a second light-emitting element. The active-matrix circuit may be voltage programmed or current programmed. The drive circuit has a pixel circuit for each pixel. The pixel circuit may include a light-emitting element, a transistor for controlling the luminous brightness of the light-emitting element, a transistor for controlling light emission timing, a capacitor for holding the gate voltage of the transistor for controlling the luminous brightness, and a transistor for GND connection without through the light-emitting element.

A light-emitting apparatus includes a display region and a peripheral region around the display region. The display region includes the pixel circuit, and the peripheral region includes a display control circuit. The mobility of a transistor constituting the pixel circuit may be smaller than the mobility of a transistor constituting the display control circuit. The gradient of the current-voltage characteristics of a transistor constituting the pixel circuit may be smaller than the gradient of the current-voltage characteristics of a transistor constituting the display control circuit. The gradient of the current-voltage characteristics can be determined by so-called Vg-Ig characteristics. A transistor constituting the pixel circuit is a transistor coupled to a light-emitting element, such as a first light-emitting element.

### [Pixel]

An organic light-emitting apparatus including an organic light-emitting element may have a plurality of pixels. Each pixel has subpixels that emit light of different colors. For example, the subpixels may have RGB emission colors.

In each pixel, a region also referred to as a pixel aperture emits light. This region is the same as the first region. The pixel aperture may be 15 µm or less or 5 µm or more. More specifically, the pixel aperture may be 11 µm, 9.5 µm, 7.4 µm, or 6.4 µm. The distance between the subpixels may be 10 µm or less, more specifically, 8 µm, 7.4 µm, or 6.4 µm.

The pixels may be arranged in a known form in a plan view. Examples include a stripe arrangement, a delta arrangement, a PenTile arrangement, and a Bayer arrangement. Each subpixel may have any known shape in a plan view. Examples include quadrangles, such as a rectangle and a rhombus, and a hexagon. As a matter of course, the rectangle also includes a figure that is not strictly rectangular but is close to rectangular. The shape of each subpixel and the pixel array can be used in combination.

### <Applications of Organic Light-Emitting Element>

The organic light-emitting element according to the present embodiment can be used as a constituent of a display apparatus or a lighting apparatus. Other applications include an exposure light source for an electrophotographic image-forming apparatus, a backlight for a liquid crystal display, and a light-emitting apparatus with a color filter in a white light source.

The display apparatus may be an image-information-processing apparatus that includes an image input unit for inputting image information from an area CCD, a linear CCD, a memory card, or the like, includes an information processing unit for processing the input information, and displays an input image on a display unit. The display apparatus may have a plurality of pixels, and at least one of the pixels may include the organic light-emitting element according to the present embodiment and a transistor coupled to the organic light-emitting element. The substrate may be a semiconductor substrate formed of silicon or the like, and the transistor may be a MOSFET formed on the substrate.

A display unit of an imaging apparatus or an inkjet printer may have a touch panel function. A driving system of the touch panel function may be, but is not limited to, an infrared radiation system, an electrostatic capacitance system, a resistive film system, or an electromagnetic induction system. The display apparatus may be used for a display unit of a multifunction printer.

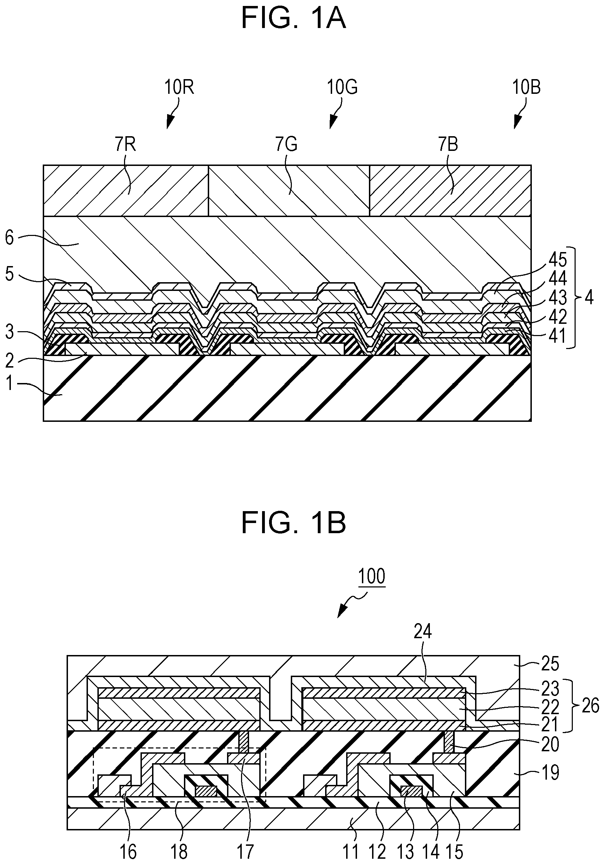

Next, a display apparatus according to the present embodiment is described with reference to the accompanying drawings. Figs. 1A and 1B are schematic cross-sectional views of an example of a display apparatus that includes an organic light-emitting element and a transistor coupled to the organic light-emitting element. The transistor is an example of an active element. The transistor may be a thin-film transistor (TFT).

Fig. 1A illustrates an example of a pixel serving as a constituent of the display apparatus according to the present embodiment. The pixel has subpixels 10. The subpixels are 10R, 10G, and 10B with different emission colors. The emission colors may be distinguished by the wavelength of light emitted from the light-emitting layer, or light emitted from each subpixel may be selectively transmitted or color-converted with a color filter or the like. Each of the subpixels 10 includes a reflective electrode serving as a first electrode 2, an insulating layer 3 covering an end of the first electrode 2, organic compound layers 4 covering the first electrode 2 and the insulating layer 3, a transparent electrode serving as a second electrode 5, a protective layer 6, and a color filter 7 on an interlayer insulating layer 1.

A transistor and/or a capacitor element may be provided under or inside the interlayer insulating layer 1. The transistor and the first electrode 2 may be electrically connected via a contact hole (not shown) or the like.

The insulating layer 3 is also referred to as a bank or a pixel separation film. The insulating layer 3 covers the ends of the first electrode 2 and surrounds the first electrode 2. A portion not covered with the insulating layer 3 is in contact with the organic compound layers 4 and serves as a light-emitting region.

The organic compound layers 4 include a hole-injection layer 41, a hole-transport layer 42, a first light-emitting layer 43, a second light-emitting layer 44, and an electron-transport layer 45.

The second electrode 5 may be a transparent electrode, a reflective electrode, or a semitransparent electrode.

The protective layer 6 reduces the penetration of moisture into the organic compound layers 4. The protective layer 6 is illustrated as a single layer but may be a plurality of layers. The protective layer 6 may include an inorganic compound layer and an organic compound layer.

The color filter 7 is divided into 7R, 7G, and 7B according to the color. The color filter 7 may be formed on a planarization film (not shown). Furthermore, a resin protective layer (not shown) may be provided on the color filter 7. The color filter 7 may be formed on the protective layer 6. Alternatively, the color filter 7 may be bonded after being provided on an opposite substrate, such as a glass substrate.

A display apparatus 100 illustrated in Fig. 1B includes an organic light-emitting element 26 and a TFT 18, which is an example of a transistor. The display apparatus 100 includes a substrate 11 made of glass, silicon, or the like and an insulating layer 12 on the substrate 11. An active element, such as the TFT 18, and a gate electrode 13, a gate-insulating film 14, and a semiconductor layer 15 of the active element are provided on the insulating layer 12. The TFT 18 has a drain electrode 16 and a source electrode 17. The TFT 18 is covered with an insulating film 19. A positive electrode 21 constituting the organic light-emitting element 26 is connected to the source electrode 17 via a contact hole 20.

Electrical connection between the electrodes of the organic light-emitting element 26 (the positive electrode 21 and a negative electrode 23) and the electrodes of the TFT 18 (the source electrode 17 and the drain electrode 16) is not limited to that illustrated in Fig. 1B. More specifically, it is only necessary to electrically connect either the positive electrode 21 or the negative electrode 23 to either the source electrode 17 or the drain electrode 16 of the TFT 18.

Although an organic compound layer 22 is a single layer in the display apparatus 100 illustrated in Fig. 1B, the organic compound layer 22 may be composed of a plurality of layers. The negative electrode 23 is covered with a first protective layer 24 and a second protective layer 25 for reducing degradation of the organic light-emitting element 26.

The transistor used as a switching element in the display apparatus 100 illustrated in Fig. 1B may be replaced with another switching element, such as a metal insulator metal (MIM) element.

The transistor used in the display apparatus 100 in Fig. 1B is not limited to a thin-film transistor including an active layer on an insulating surface of a substrate and may also be a transistor including a single crystal silicon wafer. The active layer may be single-crystal silicon, non-single-crystal silicon, such as amorphous silicon or microcrystalline silicon, or a non-single-crystal oxide semiconductor, such as indium zinc oxide or indium gallium zinc oxide. The thin-film transistor is also referred to as a TFT element.

The transistor in the display apparatus 100 of Fig. 1B may be formed within a substrate, such as a Si substrate. The phrase "formed within a substrate" means that the substrate, such as a Si substrate, itself is processed to form the transistor. Thus, the transistor within the substrate can be considered that the substrate and the transistor are integrally formed.

In the organic light-emitting element according to the present embodiment, the luminous brightness is controlled with the TFT, which is an example of a switching element. The organic light-emitting element can be provided in a plurality of planes to display an image at each luminous brightness. The switching element according to the present embodiment is not limited to the TFT and may be a transistor formed of low-temperature polysilicon or an active-matrix driver formed on a substrate, such as a Si substrate. "On a substrate" may also be referred to as "within a substrate". Whether a transistor is provided within a substrate or a TFT is used depends on the size of a display unit. For example, for an approximately 0.5-inch display unit, an organic light-emitting element can be provided on a Si substrate.

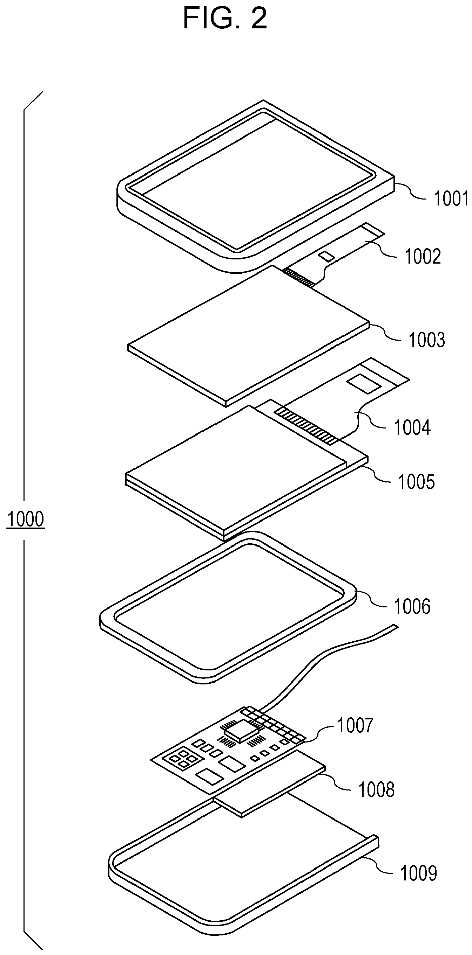

Fig. 2 is a schematic view of an example of the display apparatus according to the present embodiment. A display apparatus 1000 may include a touch panel 1003, a display panel 1005, a frame 1006, a circuit substrate 1007, and a battery 1008 between an upper cover 1001 and a lower cover 1009. The touch panel 1003 and the display panel 1005 are coupled to flexible print circuits FPC 1002 and 1004, respectively. Transistors are printed on the circuit substrate 1007. The battery 1008 is not necessarily provided when the display apparatus is not a mobile device, or may be provided at another position even when the display apparatus is a mobile device.

The display apparatus according to the present embodiment may include color filters of red, green, and blue colors. In the color filters, the red, green, and blue colors may be arranged in a delta arrangement.

The display apparatus according to the present embodiment may be used for a display unit of a mobile terminal. Such a display apparatus may have both a display function and an operation function. Examples of the mobile terminal include mobile phones, such as smartphones, tablets, and head-mounted displays.

The display apparatus according to the present embodiment may be used for a display unit of an imaging apparatus that includes an optical unit with a plurality of lenses and an imaging element for receiving light passing through the optical unit. The imaging apparatus may include a display unit for displaying information acquired by the imaging element. The display unit may be a display unit exposed outside from the imaging apparatus or a display unit located in a finder. The imaging apparatus may be a digital camera or a digital video camera.

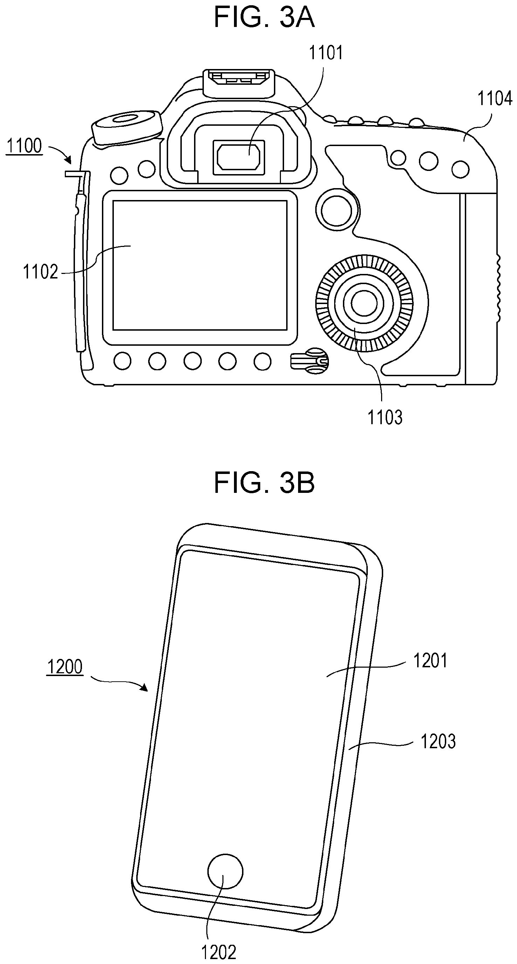

Fig. 3A is a schematic view of an example of an imaging apparatus according to the present embodiment. An imaging apparatus 1100 may include a viewfinder 1101, a rear display 1102, an operating unit 1103, and a housing 1104. The viewfinder 1101 may include the display apparatus according to the present embodiment. In such a case, the display apparatus may display environmental information, imaging instructions, and the like as well as an image to be captured. The environmental information may include the intensity of external light, the direction of external light, the travel speed of the photographic subject, the possibility that the photographic subject is shielded by a shielding material, and the like.

Because the appropriate timing for imaging is a short time, it is better to display information as soon as possible. Thus, a display apparatus including the organic light-emitting element according to the present embodiment can be used. This is because the organic light-emitting element has a high response speed. A display apparatus including the organic light-emitting element can be more suitably used than these apparatuses and liquid crystal displays that require a high display speed.

The imaging apparatus 1100 includes an optical unit (not shown). The optical unit has a plurality of lenses and focuses an image on an imaging element in the housing 1104. The focus of the lenses can be adjusted by adjusting their relative positions. This operation can also be automatically performed. The imaging apparatus may also be referred to as a photoelectric conversion apparatus. The photoelectric conversion apparatus can have, as an imaging method, a method of detecting a difference from a previous image or a method of cutting out a permanently recorded image, instead of taking an image one after another.

Fig. 3B is a schematic view of an example of electronic equipment according to the present embodiment. Electronic equipment 1200 includes a display unit 1201, an operating unit 1202, and a housing 1203. The housing 1203 may include a circuit, a printed circuit board including the circuit, a battery, and a communication unit. The operating unit 1202 may be a button or a touch panel response unit. The operating unit 1202 may be a biometric recognition unit that recognizes a fingerprint and releases the lock. Electronic equipment with a communication unit may also be referred to as communication equipment. The electronic equipment 1200 may have a lens and an imaging element and thereby further have a camera function. An image captured by the camera function is displayed on the display unit 1201. The electronic equipment 1200 may be a smartphone, a notebook computer, or the like.

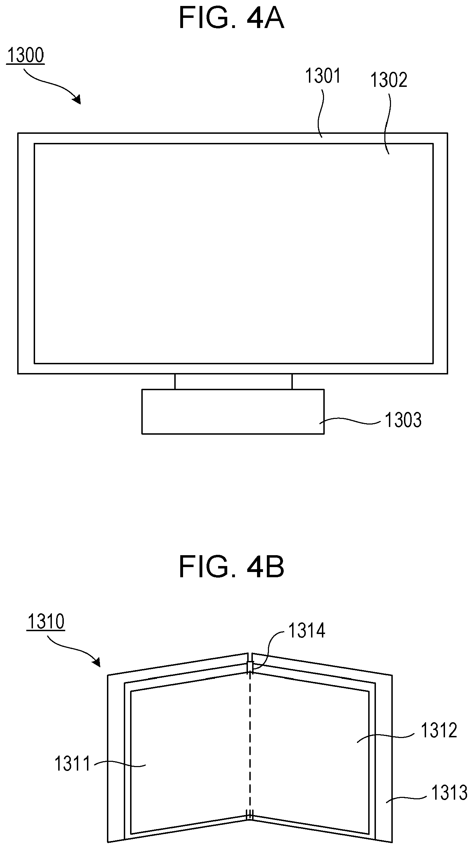

Figs. 4A and 4B are schematic views of an example of the display apparatus according to the present embodiment. Fig. 4A illustrates a display apparatus, such as a television monitor or a PC monitor. A display apparatus 1300 includes a frame 1301 and a display unit 1302. The light-emitting element according to the present embodiment may be used for the display unit 1302. The display apparatus 1300 includes a base 1303 for supporting the frame 1301 and the display unit 1302. The base 1303 is not limited to the structure illustrated in Fig. 4A. The lower side of the frame 1301 may also serve as the base. The frame 1301 and the display unit 1302 may be bent. The radius of curvature may be 5000 mm or more and 6000 mm or less.

Fig. 4B is a schematic view of another example of the display apparatus according to the present embodiment. A display apparatus 1310 in Fig. 4B is configured to be foldable and is a so-called foldable display apparatus. The display apparatus 1310 includes a first display unit 1311, a second display unit 1312, a housing 1313, and a folding point 1314. The first display unit 1311 and the second display unit 1312 may include the light-emitting element according to the present embodiment. The first display unit 1311 and the second display unit 1312 may be a single display apparatus without a joint. The first display unit 1311 and the second display unit 1312 can be divided by the folding point. The first display unit 1311 and the second display unit 1312 may display different images or one image.

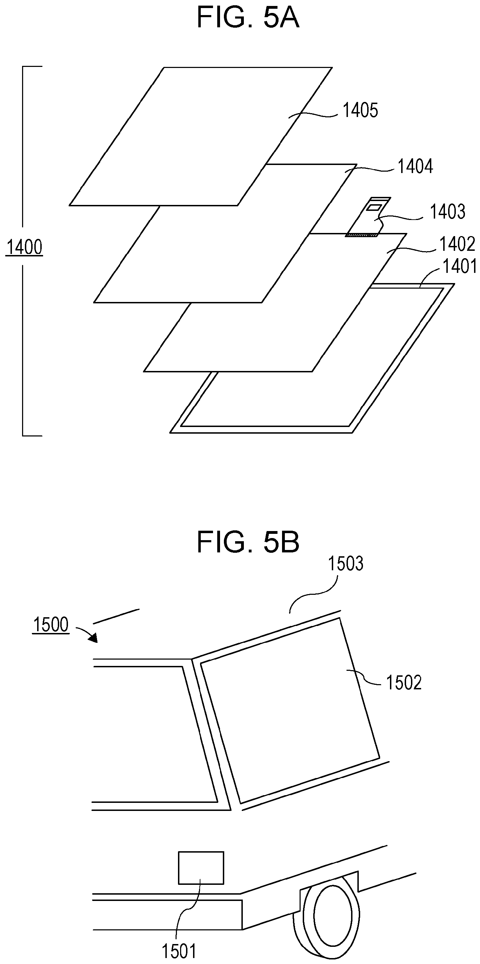

Fig. 5A is a schematic view of an example of a lighting apparatus according to the present embodiment. A lighting apparatus 1400 may include a housing 1401, a light source 1402, a circuit substrate 1403, an optical filter 1404 that transmits light emitted by the light source 1402, and a light-diffusing unit 1405. The light source 1402 may include the organic light-emitting element according to the present embodiment. The optical filter 1404 may be a filter that improves the color rendering properties of the light source. The light-diffusing unit 1405 can effectively diffuse light from the light source and widely spread light as in illumination. The optical filter 1404 and the light-diffusing unit 1405 may be provided on the light output side of the illumination. If necessary, a cover may be provided on the outermost side.

For example, the lighting apparatus is an interior lighting apparatus. The lighting apparatus may emit white light, neutral white light, or light of any color from blue to red. The lighting apparatus may have a light control circuit for controlling such light or a color control circuit for controlling emission color. The lighting apparatus may include the organic light-emitting element according to the present embodiment and a power supply circuit coupled thereto. The power supply circuit is a circuit that converts an AC voltage to a DC voltage. White has a color temperature of 4200 K, and neutral white has a color temperature of 5000 K. The lighting apparatus may have a color filter.

The lighting apparatus according to the present embodiment may include a heat dissipation unit. The heat dissipation unit releases heat from the apparatus to the outside and may be a metal or liquid silicon with a high specific heat.

Fig. 5B is a schematic view of an automobile as an example of a moving body according to the present embodiment. The automobile has a taillight as an example of a lamp. An automobile 1500 may have a taillight 1501, which comes on when a brake operation or the like is performed.

The taillight 1501 may include the organic light-emitting element according to the present embodiment. The taillight 1501 may include a protective member for protecting the organic light-emitting element. The protective member may be formed of any transparent material with moderately high strength and can be formed of polycarbonate or the like. The polycarbonate may be mixed with a furan dicarboxylic acid derivative, an acrylonitrile derivative, or the like.

The automobile 1500 may have a body 1503 and a window 1502 on the body 1503. The window 1502 may be a transparent display as long as it is not a window for checking the front and rear of the automobile. The transparent display may include the organic light-emitting element according to the present embodiment. In such a case, constituent materials, such as electrodes, of the organic light-emitting element are transparent materials.

The moving body according to the present embodiment may be a ship, an aircraft, a drone, or the like. The moving body may include a body and a lamp provided on the body. The lamp may emit light to indicate the position of the body. The lamp includes the organic light-emitting element according to the present embodiment.

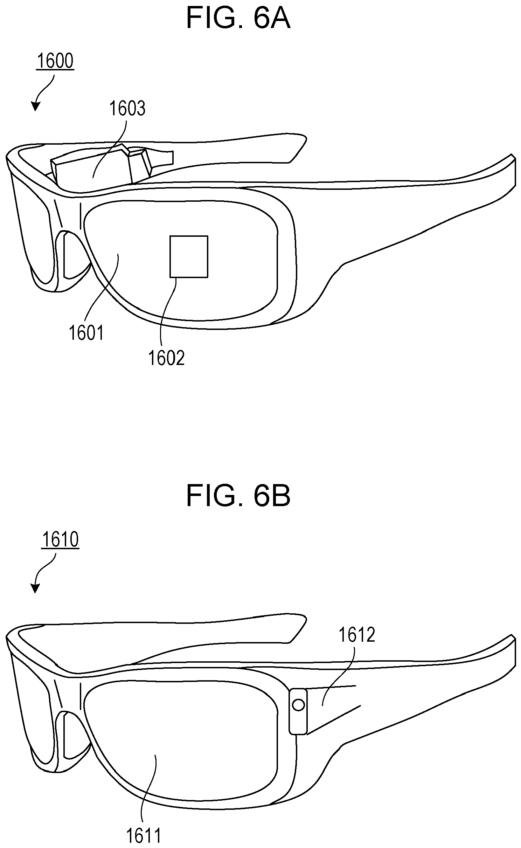

Application examples of the display apparatus according to one of the embodiments are described below with reference to Figs. 6A and 6B. The display apparatus can be applied to a system that can be worn as a wearable device, such as smart glasses, a head-mounted display (HMD), or smart contact lenses. An imaging and displaying apparatus used in such an application example includes an imaging apparatus that can photoelectrically convert visible light and a display apparatus that can emit visible light.

Fig. 6A is a schematic view of an example of a wearable device according to an embodiment of the present disclosure. Glasses 1600 (smart glasses) according to one application example are described below with reference to Fig. 6A. An imaging apparatus 1602, such as a complementary metal-oxide semiconductor (CMOS) sensor or a single-photon avalanche photodiode (SPAD), is provided on the front side of a lens 1601 of the glasses 1600. The display apparatus according to one of the embodiments is provided on the back side of the lens 1601.

The glasses 1600 further include a controller 1603. The controller 1603 functions as a power supply for supplying power to the imaging apparatus 1602 and the display apparatus. The controller 1603 controls the operation of the imaging apparatus 1602 and the display apparatus. The lens 1601 has an optical system for focusing light on the imaging apparatus 1602.

Fig. 6B is a schematic view of another example of a wearable device according to an embodiment of the present disclosure. Glasses 1610 (smart glasses) according to one application example are described below with reference to Fig. 6B. The glasses 1610 have a controller 1612, which includes an imaging apparatus corresponding to the imaging apparatus 1602 of Fig. 6A and a display apparatus. A lens 1611 includes an optical system for projecting light from the imaging apparatus of the controller 1612 and the display apparatus, and an image is projected on the lens 1611. The controller 1612 functions as a power supply for supplying power to the imaging apparatus and the display apparatus and controls the operation of the imaging apparatus and the display apparatus.

The controller 1612 may include a line-of-sight detection unit for detecting the line of sight of the wearer. Infrared radiation may be used to detect the line of sight. An infrared radiation unit emits infrared light to an eyeball of a user who is gazing at a display image. Reflected infrared light from the eyeball is detected by an imaging unit including a light-receiving element to capture an image of the eyeball. A reduction unit for reducing light from the infrared radiation unit to a display unit in a plan view is provided to reduce degradation in image quality. The line of sight of the user for the display image is detected from the image of the eyeball captured by infrared imaging. Any known technique can be applied to line-of-sight detection using the image of the eyeball. For example, it is possible to use a line-of-sight detection method based on a Purkinje image obtained by the reflection of irradiation light by the cornea. More specifically, a line-of-sight detection process based on a pupil-corneal reflection method is performed. The line of sight of the user is detected by calculating a line-of-sight vector representing the direction (rotation angle) of an eyeball on the basis of an image of a pupil and a Purkinje image included in a captured image of the eyeball using the pupil-corneal reflection method.

A display apparatus according to an embodiment of the present disclosure may include an imaging apparatus including a light-receiving element and may control a display image on the basis of line-of-sight information of a user from the imaging apparatus. More specifically, on the basis of the line-of-sight information, the display apparatus determines a first visibility region at which the user gazes and a second visibility region other than the first visibility region. The first visibility region and the second visibility region may be determined by the controller of the display apparatus or may be received from an external controller. In the display region of the display apparatus, the first visibility region may be controlled to have higher display resolution than the second visibility region. In other words, the second visibility region may have lower resolution than the first visibility region.

The display region has a first display region and a second display region different from the first display region, and the priority of the first display region and the second display region depends on the line-of-sight information. The first visibility region and the second visibility region may be determined by the controller of the display apparatus or may be received from an external controller. A region with a higher priority may be controlled to have higher resolution than another region. In other words, a region with a lower priority may have lower resolution.

The first visibility region or a region with a higher priority may be determined by artificial intelligence (AI). The AI may be a model configured to estimate the angle of the line of sight and the distance to a target ahead of the line of sight from an image of an eyeball using the image of the eyeball and the direction in which the eyeball actually viewed in the image as teaching data. The AI program may be stored in the display apparatus, the imaging apparatus, or an external device. The AI program stored in an external device is transmitted to the display apparatus via communication.

For display control based on visual recognition detection, the present disclosure can be applied to smart glasses further having an imaging apparatus for imaging the outside. Smart glasses can display captured external information in real time.

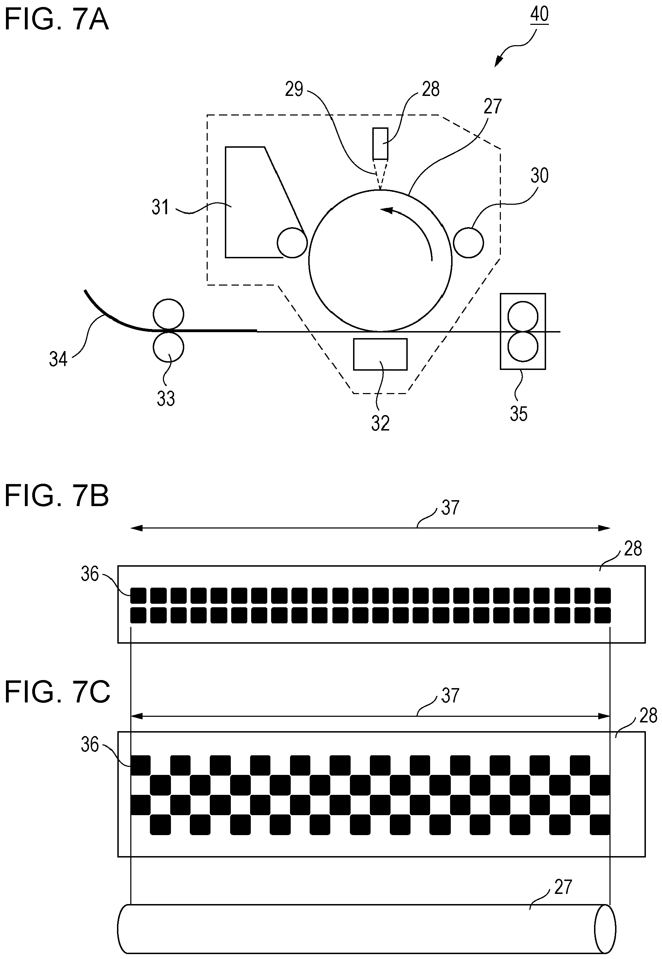

Fig. 7A is a schematic view of an example of an image-forming apparatus according to an embodiment of the present disclosure. An image-forming apparatus 40 is an electrophotographic image-forming apparatus and includes a photosensitive member 27, an exposure light source 28, a charging unit 30, a developing unit 31, a transfer unit 32, a conveying roller 33, and a fixing unit 35. The exposure light source 28 emits light 29, and an electrostatic latent image is formed on the surface of the photosensitive member 27. The exposure light source 28 includes the organic light-emitting element according to the present embodiment. The developing unit 31 contains toner and the like. The charging unit 30 electrifies the photosensitive member 27. The transfer unit 32 transfers a developed image onto a recording medium 34. The conveying roller 33 conveys the recording medium 34. The recording medium 34 is paper, for example. The fixing unit 35 fixes an image on the recording medium 34.

Figs. 7B and 7C are schematic views of the exposure light source 28, wherein a plurality of light-emitting portions 36 are arranged on a long substrate. An arrow 37 indicates a direction parallel to the axis of the photosensitive member and indicates a column direction in which the organic light-emitting elements are arranged. This column direction is the same as the direction of the rotation axis of the photosensitive member 27. This direction can also be referred to as the major-axis direction of the photosensitive member 27. In Fig. 7B, the light-emitting portions 36 are arranged in the major-axis direction of the photosensitive member 27. In Fig. 7C, unlike Fig. 7B, the light-emitting portions 36 are alternately arranged in the column direction in the first and second columns. The first and second columns are arranged at different positions in the row direction. In the first column, the light-emitting portions 36 are arranged at intervals. In the second column, the light-emitting portions 36 are arranged at positions corresponding to the spaces between the light-emitting portions 36 of the first column. Thus, the light-emitting portions 36 are also arranged at intervals in the row direction. The arrangement in Fig. 7C can also be referred to as a grid-like pattern, a staggered pattern, or a checkered pattern, for example.

As described above, an apparatus including the organic light-emitting element according to the present embodiment can be used to stably display a high-quality image for extended periods.

### EXAMPLES

The present disclosure is described below with exemplary embodiments. However, the present disclosure is not limited these exemplary embodiments.

### [Exemplary Embodiment 1 (Synthesis of Exemplary Compound A3)]

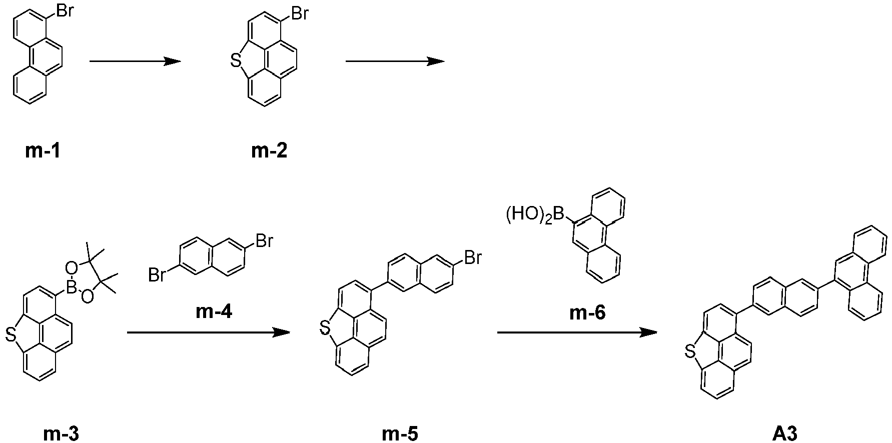

### (1) Synthesis of Compound m-2

A 500-ml recovery flask was charged with the following reagent and solvent.
Compound m-1: 8.2 g (0.032 mol)
Hexane: 160 ml

The reaction solution was then stirred in a nitrogen atmosphere at -30°C, and TMEDA (24 ml, 0.16 mol) and n-BuLi (1.6 M, 100 ml, 0.16 mol) were added to the reaction solution from the dropping funnel. After stirring in a nitrogen atmosphere for 3 hours, S₂Cl₂ (6.4 ml, 0.08 mol) was slowly added and allowed to react at normal temperature for 24 hours. The reaction mixture was subjected to liquid separation with water and chloroform and was then purified by silica gel column chromatography (developing liquid: heptane/chloroform) to yield 5.0 g of a compound m-2 (yield: 55%).

### (2) Synthesis of Compound m-3

A 300-ml recovery flask was charged with the following reagents and solvents.
Compound m-2: 2.5 g (8.7 mmol)
Bispinacolato borane: 2.3 g (8.7 mmol)
[1,1'-bis(diphenylphosphino)ferrocene]palladium(II) dichloride dichloromethane adduct: 0.57 g (0.71 mmol)
Potassium acetate: 2.3 g (22.6 mmol)
1,4-dioxane: 90 ml

The reaction solution was heated under reflux with stirring in a nitrogen atmosphere for 5 hours. After completion of the reaction, the solvent was distilled off under reduced pressure, and the resulting solid was purified with a silica gel column (chloroform:heptane = 2:1) to yield 2.0 g of m-3 (yield: 70%).

### (3) Synthesis of Compound m-5

A 300-ml recovery flask was charged with the following reagents and solvents.
Compound m-3: 1.5 g (4.5 mmol)
Compound m-4: 1.3 g (4.5 mmol)
Pd(PPh₃)₄: 0.26 g (0.22 mmol)
Toluene: 45 ml
Ethanol: 23 ml
2 M aqueous sodium carbonate: 23 ml

The reaction solution was then heated and stirred under reflux in a nitrogen stream for 6 hours. After completion of the reaction, the product was subjected to liquid separation with water and toluene, was purified by column chromatography (toluene:heptane), and was then recrystallized from toluene to yield 1.5 g of a compound m-5 as a white solid (yield: 80%).

### (4) Synthesis of Exemplary Compound A3

A 300-ml recovery flask was charged with the following reagents and solvents.
Compound m-5: 1.0 g (2.4 mmol)
Compound m-6: 1.3 g (2.4 mmol)
Pd(PPh₃)₄: 0.14 g (0.12 mmol)
Toluene: 30 ml
Ethanol: 15 ml
2 M aqueous sodium carbonate: 15 ml

The reaction solution was then heated and stirred under reflux in a nitrogen stream for 6 hours. After completion of the reaction, the product was subjected to liquid separation with water and toluene, was purified by column chromatography (toluene:heptane), and was then recrystallized from toluene to yield 1.1 g of a compound A3 as a white solid (yield: 90%).

The exemplary compound A3 was subjected to mass spectrometry with
MALDI-TOF-MS (Autoflex LRF manufactured by Bruker).
[MALDI-TOF-MS]
Measured value: m/z = 510 Calculated value: C₃₈H₂₂S = 510

### [Exemplary Embodiments 2 to 30 (Synthesis of Exemplary Compounds)]

Exemplary compounds were synthesized in the same manner as in Exemplary Embodiment 1 except that the raw materials m-1, m-4, and m-6 were changed as shown in Tables 3 to 6. Actual values m/z measured by mass spectrometry in the same manner as in Exemplary Embodiment 1 are also shown.

**Table 3**

| Exemplary embodiment | Exemplary embodiment | Raw material m-1 | Raw material m-4 | Raw material m-6 | m/z |
|---|---|---|---|---|---|
| Exemplary embodiment 2 | A1 | 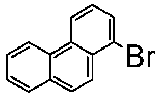 | 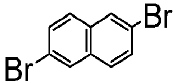 | 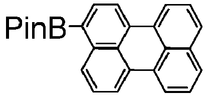 | 584 |
| Exemplary embodiment 3 | A4 | 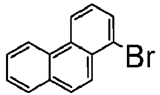 | 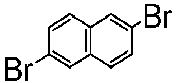 | 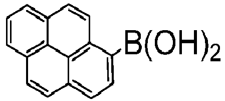 | 534 |
| Exemplary embodiment 4 | A9 | 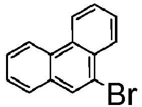 | 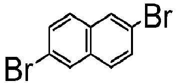 | 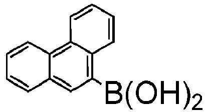 | 510 |
| Exemplary embodiment 5 | A10 | 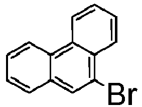 | 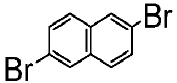 | 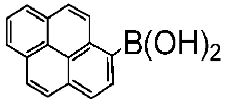 | 534 |
| Exemplary embodiment 6 | A12 | 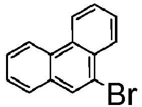 | 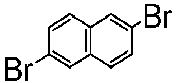 | 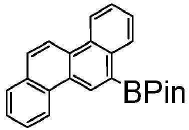 | 560 |
| Exemplary embodiment 7 | A15 | 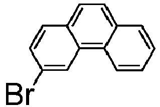 | 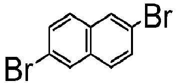 | 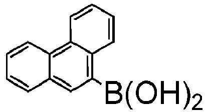 | 510 |
| Exemplary embodiment 8 | A16 | 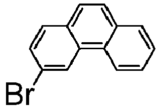 | 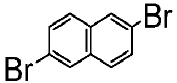 | 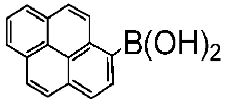 | 534 |
| Exemplary embodiment 9 | A23 | 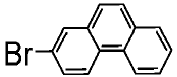 | 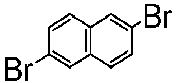 | 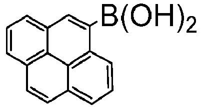 | 534 |

**Table 4**

| Exemplary embodiment | Exemplary embodiment | Raw material m-1 | Raw material m-4 | Raw material m-6 | m/z |
|---|---|---|---|---|---|
| Exemplary embodiment 10 | A25 | 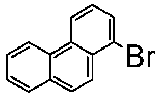 | 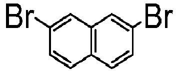 | 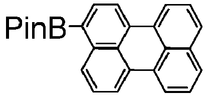 | 584 |
| Exemplary embodiment 11 | A28 | 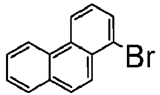 | 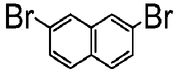 | 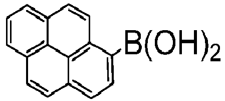 | 534 |
| Exemplary embodiment 12 | A29 | 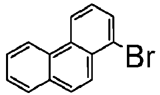 | 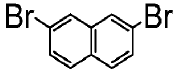 | 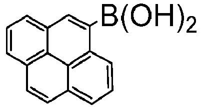 | 534 |
| Exemplary embodiment 13 | A33 | 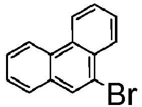 | 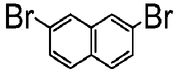 | 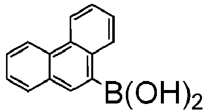 | 510 |
| Exemplary embodiment 14 | A34 | 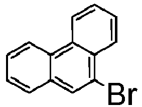 | 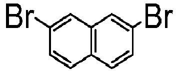 | 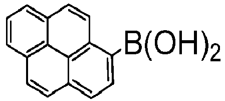 | 534 |
| Exemplary embodiment 15 | A40 | 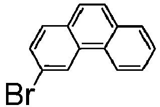 | 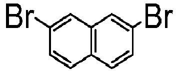 | 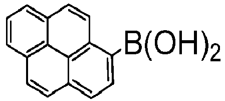 | 534 |
| Exemplary embodiment 16 | A41 | 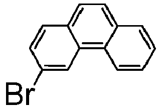 | 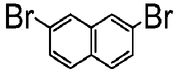 | 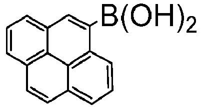 | 534 |

**Table 5**

| Exemplary embodiment | Exemplary embodiment | Raw material m-1 | Raw material m-4 | Raw material m-6 | m/z |
|---|---|---|---|---|---|
| Exemplary embodiment 17 | A43 | 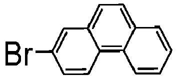 | 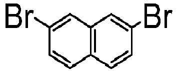 | 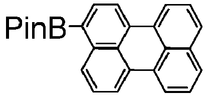 | 584 |
| Exemplary embodiment 18 | A46 | 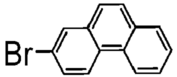 | 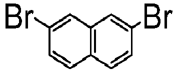 | 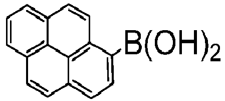 | 534 |
| Exemplary embodiment 19 | B1 | 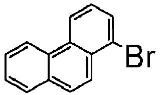 | 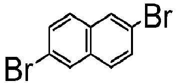 | 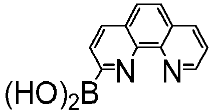 | 512 |
| Exemplary embodiment 20 | B14 | 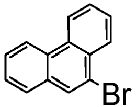 | 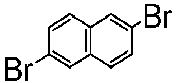 | 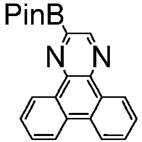 | 562 |
| Exemplary embodiment 21 | B17 | 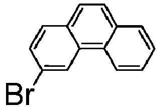 | 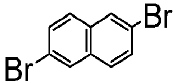 | 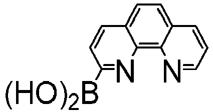 | 512 |
| Exemplary embodiment 22 | B21 | 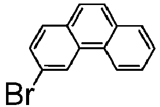 | 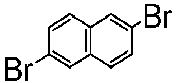 | 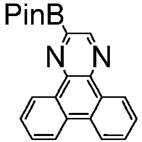 | 562 |
| Exemplary embodiment 23 | B23 | 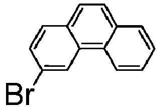 | 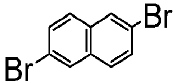 | 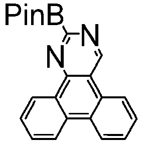 | 562 |
| Exemplary embodiment 24 | B25 | 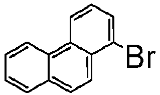 | 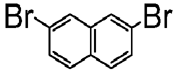 | 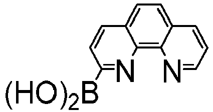 | 512 |

**Table 6**

| Exemplary embodiment | Exemplary embodiment | Raw material m-1 | Raw material m-4 | Raw material m-6 | m/z |
|---|---|---|---|---|---|
| Exemplary embodiment 25 | B29 | 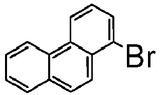 | 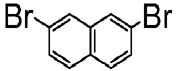 | 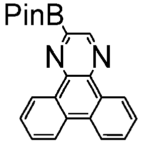 | 562 |
| Exemplary embodiment 26 | B31 | 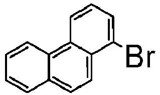 | 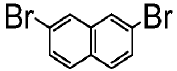 | 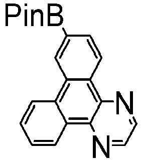 | 562 |
| Exemplary embodiment 27 | B36 | 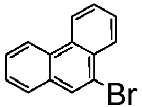 | 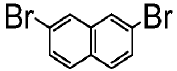 | 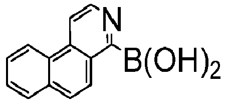 | 511 |
| Exemplary embodiment 28 | B41 | 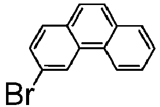 | 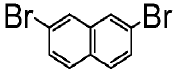 | 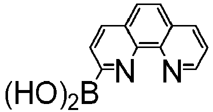 | 512 |
| Exemplary embodiment 29 | C7 | 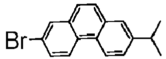 | 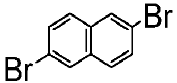 | 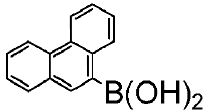 | 552 |
| Exemplary embodiment 30 | C14 | 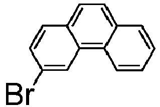 | 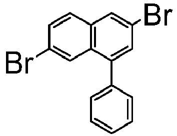 | 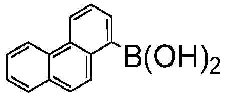 | 586 |

### [Exemplary Embodiment 31]

An organic light-emitting element of a bottom emission type was produced. The organic light-emitting element included a positive electrode, a hole-injection layer, a hole-transport layer, an electron-blocking layer, a light-emitting layer, a hole-blocking layer, an electron-transport layer, an electron-injection layer, and a negative electrode sequentially formed on a substrate.

First, an ITO film was formed on a glass substrate and was subjected to desired patterning to form an ITO electrode (positive electrode). The ITO electrode had a thickness of 100 nm. The substrate on which the ITO electrode was formed was used as an ITO substrate in the following process. Vacuum deposition was then performed by resistance heating in a vacuum chamber at 1.33 × 10⁻⁴ Pa to continuously form an organic compound layer and an electrode layer shown in Table 7 on the ITO substrate. The counter electrode (a metal electrode layer, a negative electrode) had an electrode area of 3 mm².

**Table 7**

| | Material | | | Film thickness (nm) |
|---|---|---|---|---|
| Negative electrode | Al | | | 100 |
| Electron-injection layer (EIL) | LiF | | | 1 |
| Electron-transport layer (ETL) | ET2 | | | 20 |
| Hole-blocking layer (HBL) | ET11 | | | 20 |
| Light-emitting layer (EML) | Host | A3 | Weight ratio A3:BD8 =98:2 | 20 |
| | Guest | BD8 | | |
| Electron-blocking layer (EBL) | HT19 | | | 15 |
| Hole-transport layer (HTL) | HT3 | | | 30 |
| Hole-injection layer (HIL) | HT16 | | | 5 |

The characteristics of the element were measured and evaluated. The emission color of the light-emitting element was good blue light emission. The maximum external quantum efficiency (E.Q.E.) was 1.3 on the assumption that the maximum external quantum efficiency (E.Q.E.) of Comparative Example 1 was 1.0. A continuous operation test was performed at a current density of 100 mA/cm² to measure the time when the luminance decay rate reached 5%. The present exemplary embodiment had a luminance decay rate ratio of 1.4 on the assumption that the time when the luminance decay rate of Comparative Example 1 reached 5% was 1.0.

In the present exemplary embodiment, with respect to measuring apparatuses, more specifically, the current-voltage characteristics were measured with a microammeter 4140B manufactured by Hewlett-Packard Co., and the luminous brightness was measured with BM7 manufactured by Topcon Corporation.

### [Exemplary Embodiments 32 to 40 and Comparative Example 1]

Organic light-emitting elements were produced in the same manner as in Exemplary Embodiment 31 except that the compounds shown in Table 8 were used. The host material used in Comparative Example 1 is Compound 1-A described in Patent Literature 1. Characteristics of the elements were measured and evaluated in the same manner as in Exemplary Embodiment 31. Table 8 shows the measurement results.

**Table 8**

| | HIL | HTL | EBL | EML | | HBL | ETL | External quantum efficiency ratio | Luminance decay rate ratio |
|---|---|---|---|---|---|---|---|---|---|
| | | | | Host | Guest | | | | |
| Exemplary embodiment 32 | HT16 | HT2 | HT15 | A9 | B7 | ET11 | ET2 | 1.6 | 1.1 |
| Exemplary embodiment 33 | HT16 | HT2 | HT15 | A12 | BD8 | ET12 | ET2 | 1.2 | 1.3 |
| Exemplary embodiment 34 | HT16 | HT2 | HT15 | A15 | BD10 | ET11 | ET15 | 1.2 | 1.2 |
| Exemplary embodiment 35 | HT16 | HT3 | HT19 | A16 | BD10 | ET11 | ET2 | 1.6 | 1.4 |
| Exemplary embodiment 36 | HT16 | HT2 | HT19 | A28 | BD7 | ET12 | ET2 | 1.7 | 1.3 |
| Exemplary embodiment 37 | HT16 | HT3 | HT15 | A33 | BD8 | ET11 | ET15 | 1.5 | 1.2 |
| Exemplary embodiment 38 | HT16 | HT2 | HT19 | A34 | BD8 | ET12 | ET2 | 1.2 | 1.2 |
| Exemplary embodiment 39 | HT16 | HT3 | HT15 | A40 | BD7 | ET12 | ET2 | 1.4 | 1.4 |
| Exemplary embodiment 40 | HT16 | HT3 | HT19 | A46 | BD8 | ET11 | ET15 | 1.5 | 1.3 |
| | | | | | | | | | |
| Comparative example 1 | HT16 | HT3 | HT15 | Comparative compound 1-A | BD8 | ET11 | ET2 | 1.0 | 1.0 |

Table 8 shows that the light-emitting elements of the exemplary embodiments had maximum external quantum efficiency (E.Q.E.) higher than 1.0 of Comparative Example 1 and had higher light emission efficiency. Furthermore, the light-emitting elements of the exemplary embodiments had a longer life. This is because the organic compound according to the present embodiment has good film properties, high sublimability, and high long-term operation durability.

### [Exemplary Embodiments 41 to 49 and Comparative Example 2]

Organic light-emitting elements were produced in the same manner as in Exemplary Embodiment 31 except that the guest material was changed to GD6 and the compounds shown in Table 9 were used. The host material used in Comparative Example 2 is Compound 1-A described in Patent Literature 1. Characteristics of the elements were measured and evaluated in the same manner as in Exemplary Embodiment 31. Table 9 shows the measurement results.

The emission colors of the light-emitting elements were good green light emission. The maximum external quantum efficiency (E.Q.E.) is the relative ratio to the maximum external quantum efficiency (E.Q.E.) (1.0) of Comparative Example 2. The luminance decay rate ratio is a relative ratio to the time (1.0) when the luminance decay rate of Comparative Example 2 reaches 5%.

**Table 9**

| | HIL | HTL | EBL | EML | | HBL | ETL | External quantum efficiency ratio | Luminance decay rate ratio |
|---|---|---|---|---|---|---|---|---|---|
| | | | | Host | Guest | | | | |
| Exemplary embodiment 41 | HT16 | HT2 | HT15 | B14 | GD6 | ET11 | ET2 | 1.6 | 1.9 |
| Exemplary embodiment 42 | HT16 | HT3 | HT19 | B17 | GD6 | ET12 | ET15 | 1.2 | 1.7 |
| Exemplary embodiment 43 | HT16 | HT2 | HT15 | B21 | GD6 | ET11 | ET2 | 1.2 | 1.8 |
| Exemplary embodiment 44 | HT16 | HT2 | HT19 | B25 | GD6 | ET11 | ET2 | 1.6 | 1.6 |
| Exemplary embodiment 45 | HT16 | HT3 | HT19 | B29 | GD6 | ET12 | ET15 | 1.7 | 1.9 |
| Exemplary embodiment 46 | HT16 | HT3 | HT19 | B31 | GD6 | ET12 | ET15 | 1.5 | 1.7 |
| Exemplary embodiment 47 | HT16 | HT3 | HT19 | B41 | GD6 | ET11 | ET15 | 1.2 | 1.8 |
| Exemplary embodiment 48 | HT16 | HT2 | HT15 | C7 | GD6 | A1 | ET2 | 1.3 | 1.4 |
| Exemplary embodiment 49 | HT16 | HT2 | HT15 | C14 | GD6 | A4 | ET15 | 1.2 | 1.4 |
| | | | | | | | | | |
| Comparative example 2 | HT16 | HT3 | HT15 | Comparative compound 1-A | GD6 | ET11 | ET2 | 1.0 | 1.0 |

Table 9 shows that the light-emitting elements of the exemplary embodiments had maximum external quantum efficiency (E.Q.E.) higher than 1.0 of Comparative Example 2 and had higher light emission efficiency. Furthermore, the light-emitting elements of the exemplary embodiments had a longer life. This is because the organic compound according to the present embodiment has good film properties, high sublimability, and high long-term operation durability.

### [Exemplary Embodiments 50 to 52 and Comparative Example 3]

Organic light-emitting elements were produced in the same manner as in Exemplary Embodiment 31 except that the guest material was changed to RD1 and the compounds shown in Table 10 were used. The host material used in Comparative Example 3 is Compound 1-A described in Patent Literature 1. Characteristics of the elements were measured and evaluated in the same manner as in Exemplary Embodiment 31. Table 10 shows the measurement results.

The emission color of the light-emitting element was good red light emission. The maximum external quantum efficiency (E.Q.E.) is the relative ratio to the maximum external quantum efficiency (E.Q.E.) (1.0) of Comparative Example 3. The luminance decay rate ratio is a relative ratio to the time (1.0) when the luminance decay rate of Comparative Example 3 reaches 5%.

**Table 10**

| | HIL | HTL | EBL | EML | | HBL | ETL | External quantum efficiency ratio | Luminance decay rate ratio |
|---|---|---|---|---|---|---|---|---|---|
| | | | | Host | Guest | | | | |
| Exemplary embodiment 50 | HT16 | HT2 | HT19 | A1 | RD1 | ET11 | ET2 | 1.6 | 2.0 |
| Exemplary embodiment 51 | HT16 | HT3 | HT15 | A25 | RD1 | ET12 | ET15 | 1.4 | 1.7 |
| Exemplary embodiment 52 | HT16 | HT3 | HT19 | A43 | RD1 | ET11 | ET2 | 1.2 | 1.6 |
| | | | | | | | | | |
| Comparative example 3 | HT16 | HT3 | HT15 | Comparative compound 1-A | RD1 | ET11 | ET2 | 1.0 | 1.0 |

Table 10 shows that the light-emitting elements of the exemplary embodiments had maximum external quantum efficiency (E.Q.E.) higher than 1.0 of Comparative Example 3 and had higher light emission efficiency. Furthermore, the light-emitting elements of the exemplary embodiments had a longer life. This is because the organic compound according to the present embodiment has good film properties, high sublimability, and high long-term operation durability.

### [Exemplary Embodiment 53]

An organic light-emitting element was produced in the same manner as in Exemplary Embodiment 31 except that the organic compound layer and the electrode layer shown in Table 11 were continuously formed.

**Table 11**

| | Material | | | Film thickness (nm) |
|---|---|---|---|---|
| Negative electrode | Al | | | 100 |
| Electron-injection layer (EIL) | LiF | | | 1 |
| Electron-transport layer (ETL) | ET2 | | | 20 |
| Hole-blocking layer (HBL) | ET11 | | | 20 |
| Second light-emitting layer (EML2) | Host | EM1 | Weight ratio EM1:BD8:A3 =60:10:30 | 20 |
| | Guest | BD8 | | |
| | Assist | A3 | | |
| First light-emitting layer (EML1) | Host | EM1 | Weight ratio EM1:RD1:GD6 =97.2:0.3:2.5 | 20 |
| | Guest 1 | RD1 | | |
| | Guest 2 | GD6 | | |
| Electron-blocking layer (EBL) | HT19 | | | 15 |
| Hole-transport layer (HTL) | HT3 | | | 30 |
| Hole-injection layer (HIL) | HT16 | | | 5 |

Characteristics of the element were measured and evaluated in the same manner as in Exemplary Embodiment 31. The light-emitting element had a white emission color and had a luminance decay rate ratio of 1.8 on the assumption that the time when the luminance decay rate of Comparative Example 4 reached 5% was 1.0.

### [Exemplary Embodiments 54 to 70]

Organic light-emitting elements were produced in the same manner as in Exemplary Embodiment 53 except that the compounds (upper row) and the mass ratios (lower row) shown in Tables 12 and 13 were used. Characteristics of the elements were measured and evaluated in the same manner as in Exemplary Embodiment 53. Tables 12 and 13 show the measurement results.

**Table 12**

| | EML | | | EML | | | Luminance decay rate ratio |
|---|---|---|---|---|---|---|---|
| | Host | Guest | Assist | Host | Guest 1 | Guest 2 | |
| Exemplary embodiment 54 | EM1 70% | BD6 2% | A16 28% | EM1 97.2 | RD1 0.3 | GD6 2.5 | 1.8 |
| Exemplary embodiment 55 | EM2 70% | BD6 2% | A33 28% | EM2 97.2 | RD1 0.3 | GD6 2.5 | 1.6 |
| Exemplary embodiment 56 | EM4 70% | BD6 2% | A33 28% | EM4 97.2 | RD1 0.3 | GD6 2.5 | 1.7 |
| Exemplary embodiment 57 | EM27 70% | BD6 2% | A33 28% | EM27 97.2 | RD1 0.3 | GD6 2.5 | 1.5 |
| Exemplary embodiment 58 | EM41 70% | BD6 2% | A28 28% | EM41 97.2 | RD1 0.3 | GD6 2.5 | 1.8 |
| Exemplary embodiment 59 | EM27 96% | BD6 2% | A33 2% | EM27 97.2 | RD1 0.3 | GD6 2.5 | 1.7 |
| Exemplary embodiment 60 | EM27 97.5% | BD6 2% | A33 0.5% | EM27 97.2 | RD1 0.3 | GD6 2.5 | 1.5 |
| Exemplary embodiment 61 | EM41 70% | BD6 2% | A28 2% | EM41 97.2 | RD1 0.3 | GD6 2.5 | 1.5 |
| Exemplary embodiment 62 | EM41 70% | BD6 2% | A28 0.5% | EM41 97.2 | RD1 0.3 | GD6 2.5 | 1.5 |
| Exemplary embodiment 63 | EM42 70% | BD6 2% | A34 2% | EM42 97.2 | RD1 0.3 | GD6 2.5 | 1.8 |
| Exemplary embodiment 64 | EM42 70% | BD6 2% | A40 0.5% | EM42 97.2 | RD1 0.3 | GD6 2.5 | 1.9 |
| Exemplary embodiment 65 | EM42 70% | BD6 2% | A46 2% | EM42 97.2 | RD1 0.3 | GD6 2.5 | 1.5 |
| Exemplary embodiment 66 | EM42 70% | BD6 2% | A46 0.5% | EM42 97.2 | RD1 0.3 | GD6 2.5 | 1.7 |
| Exemplary embodiment 67 | EM41 70% | BD6 2% | A34 2% | EM41 97.2 | RD1 0.3 | GD6 2.5 | 1.5 |
| Exemplary embodiment 68 | EM41 70% | BD6 2% | A40 0.5% | EM41 97.2 | RD1 0.3 | GD6 2.5 | 1.5 |
| Exemplary embodiment 69 | EM41 70% | BD6 2% | A46 2% | EM41 97.2 | RD1 0.3 | GD6 2.5 | 1.5 |
| Exemplary embodiment 70 | EM41 70% | BD6 2% | A46 0.5% | EM41 97.2 | RD1 0.3 | GD6 2.5 | 1.5 |
| | | | | | | | |
| Comparative example 4 | EM1 70% | BD6 2% | - | EM1 97.2 | RD1 0.3 | GD6 2.5 | 1.0 |
| Comparative example 5 | EM1 70% | BD6 2% | EM13 2% | EM1 97.2 | RD1 0.3 | GD6 2.5 | 0.8 |

**Table 13**

| | EML2 | | | EML1 | | | Luminance decay rate ratio |
|---|---|---|---|---|---|---|---|
| | Host | Guest | Assist | Host | Guest | Assist | |
| Exemplary embodiment 63 | EM42 96 | BD6 2 | A34 2 | EM42 97.2 | RD1 0.3 | GD6 2.5 | 1.8 |
| Exemplary embodiment 64 | EM42 97.5 | BD6 2 | A40 0.5 | EM42 97.2 | RD1 0.3 | GD6 2.5 | 1.9 |
| Exemplary embodiment 65 | EM42 96 | BD6 2 | A46 2 | EM42 97.2 | RD1 0.3 | GD6 2.5 | 1.5 |
| Exemplary embodiment 66 | EM42 97.5 | BD6 2 | A46 0.5 | EM42 97.2 | RD1 0.3 | GD6 2.5 | 1.7 |
| Exemplary embodiment 67 | EM41 96 | BD6 2 | A34 2 | EM41 97.2 | RD1 0.3 | GD6 2.5 | 1.5 |
| Exemplary embodiment 68 | EM41 97.5 | BD6 2 | A40 0.5 | EM41 97.2 | RD1 0.3 | GD6 2.5 | 1.5 |
| Exemplary embodiment 69 | EM41 96 | BD6 2 | A46 2 | EM41 97.2 | RD1 0.3 | GD6 2.5 | 1.5 |
| Exemplary embodiment 70 | EM41 97.5 | BD6 2 | A46 0.5 | EM41 97.2 | RD1 0.3 | GD6 2.5 | 1.5 |
| Comparative example 4 | EM1 98 | BD6 2 | - | EM1 97.2 | RD1 0.3 | GD6 2.5 | 1.0 |
| Comparative example 5 | EM1 96 | BD6 2 | EM13 2 | EM1 97.2 | RD1 0.3 | GD6 2.5 | 0.8 |

The present disclosure can provide an organic compound that can be used for an organic light-emitting element with good element life characteristics.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

The present disclosure discloses an organic compound represented by the following general formula [1]:

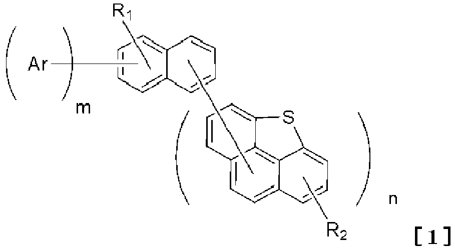

Ar is selected from the group consisting of a substituted or unsubstituted aryl group and a substituted or unsubstituted heteroaryl group. R₁ and R₂ are independently selected from the group consisting of a hydrogen atom and a substituent. m and n are selected from integers of 1 or more and 3 or less.

## Claims

1. An organic compound represented by the following general formula [1]: 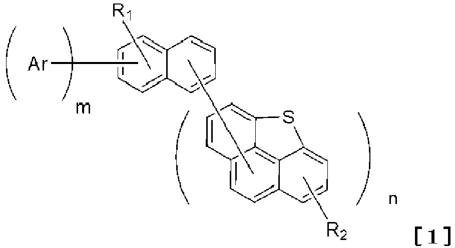
wherein Ar is selected from the group consisting of a substituted or unsubstituted aryl group and a substituted or unsubstituted heteroaryl group,
R₁ and R₂ are independently selected from the group consisting of a hydrogen atom, a deuterium atom, a halogen atom, a substituted or unsubstituted alkyl group, a substituted or unsubstituted alkoxy group, a substituted or unsubstituted amino group, a substituted or unsubstituted silyl group, a substituted or unsubstituted aryl group, a substituted or unsubstituted heteroaryl group, a substituted or unsubstituted aryloxy group, a substituted or unsubstituted heteroaryloxy group, and a cyano group, and
m and n are selected from integers of 1 or more and 3 or less.

2. The organic compound according to Claim 1, wherein Ar denotes a fused ring.

3. The organic compound according to Claim 2, wherein Ar denotes a fused ring of three or more 5-membered or 6-membered rings.

4. The organic compound according to Claim 3, wherein Ar is selected from the group consisting of the following [a] to [n]: 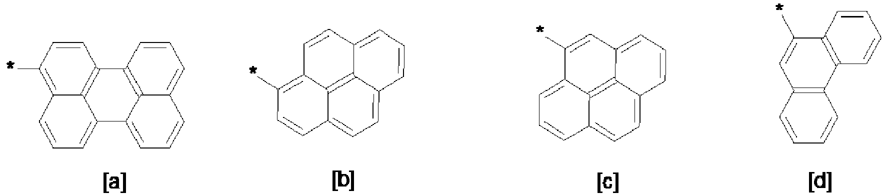 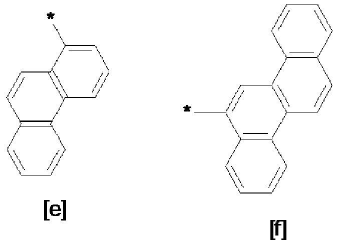 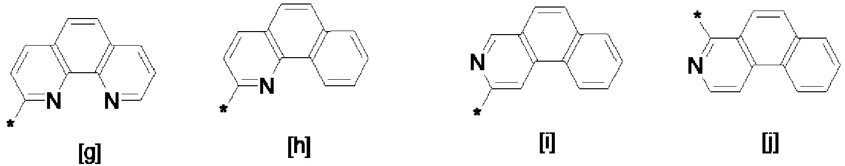 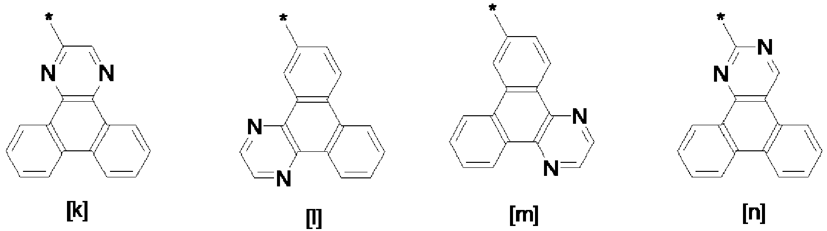 wherein ^{∗} represents a binding position to a naphthalene ring.

5. The organic compound according to Claim 4, wherein Ar is selected from the group consisting of [a] to [f].

6. An organic light-emitting element comprising:
a first electrode (2);
a second electrode (5); and
one of more organic compound layers (4) between the first electrode and the second electrode,
wherein at least one layer of the organic compound layers contains the organic compound according to any one of Claims 1 to 5.

7. The organic light-emitting element according to Claim 6, wherein the layer containing the organic compound is a light-emitting layer.

8. The organic light-emitting element according to Claim 6 or 7, wherein the light-emitting layer further comprises a light-emitting material with lower lowest unoccupied molecular orbital energy than the organic compound.

9. The organic light-emitting element according to Claim 8, wherein the light-emitting material has a 5-membered ring in its molecular structure.

10. The organic light-emitting element according to Claim 7, wherein the light-emitting layer further comprises a light-emitting material with higher highest occupied molecular orbital energy than the organic compound.

11. The organic light-emitting element according to Claim 7, wherein the light-emitting layer further comprises a second component.

12. The organic light-emitting element according to Claim 11, wherein the second component has higher highest occupied molecular orbital energy than the organic compound.

13. The organic light-emitting element according to Claim 11, wherein the second component has two or more fused rings of either a phenanthrene ring or a pyrene ring in its molecule.

14. The organic light-emitting element according to Claim 11, wherein a second component content with respect to a total of the organic compound and the second component is 10% by mass or less.

15. The organic light-emitting element according to Claim 7, further comprising another light-emitting layer on the light-emitting layer, wherein the other light-emitting layer emits light of a different color from the light-emitting layer.

16. A display apparatus comprising a plurality of pixels, wherein at least one of the plurality of pixels includes the organic light-emitting element according to any one of Claims 6 to 15 and a transistor coupled to the organic light-emitting element.

17. A photoelectric conversion apparatus comprising:
an optical unit including a plurality of lenses;
an imaging element configured to receive light passing through the optical unit; and
a display unit (1201, 1302) configured to display an image taken by the imaging element,
wherein the display unit includes the organic light-emitting element according to any one of Claims 6 to 15.

18. Electronic equipment comprising:
a display unit including the organic light-emitting element according to any one of Claims 6 to 15;
a housing (1104, 1203, 1313, 1401) in which the display unit is provided; and
a communication unit provided in the housing and configured to communicate with an outside.

19. Alighting apparatus comprising:
a light source (1402) including the organic light-emitting element according to any one of Claims 6 to 15; and
a light-diffusing unit (1405) or an optical filter (1404) configured to transmit light emitted by the light source.

20. A moving body comprising:
a lamp including the organic light-emitting element according to any one of Claims 6 to 15; and
a body (1503) to which the lamp is provided.
